# EUROPEAN PATENT APPLICATION

(11) **EP 2 851 089 A1**
(43) Date of publication of application: **25.03.2015**
(21) Application number: 13185827.6
(22) Date of filing: 24.09.2013
(51) Int. Cl.: A61K 39/39, A61K 47/48, C07K 14/28

(54) **Cholera toxin a-like polypeptide useful as adjuvant component**

(71) Applicant: Gotovax AB, 426 74 Västra Frölunda (SE)
(72) Inventor: Holmgren, Jan, 426 74 Västra Frölunda (SE); Lebens, Michael, 523 99 Hökerum (SE)
(74) Representative: Novitas Patent AB

(57) **Abstract**

A cholera toxin-like polypeptide useful as adjuvant is provided. Polynucleotide coding to the polypeptide and associated vectors, host cells and methods of production are provided. Adjuvants, compositions comprising the polypeptide, and uses thereof are also provided.

SEQUENCE LISTING

<110>  Gotovax AB

<120>  CHOLERA TOXIN A-LIKE POLYPEPTIDE USEFUL AS ADJUVANT COMPONENT

<130>  P41304703SE00

<160>  16

<170>  PatentIn version 3.5

<210>  1
<211>  240
<212>  PRT
<213>  Vibrio cholerae

<400>  1

Asn Asp Asp Lys Leu Tyr Arg Ala Asp Ser Arg Pro Pro Asp Glu Ile
1               5                   10                  15

Lys Gln Ser Gly Gly Leu Met Pro Arg Gly Gln Ser Glu Tyr Phe Asp
            20                  25                  30

Arg Gly Thr Gln Met Asn Ile Asn Leu Tyr Asp His Ala Arg Gly Thr
        35                  40                  45

Gln Thr Gly Phe Val Arg His Asp Asp Gly Tyr Val Ser Thr Ser Ile
    50                  55                  60

Ser Leu Arg Ser Ala His Leu Val Gly Gln Thr Ile Leu Ser Gly His
65                  70                  75                  80

Ser Thr Tyr Tyr Ile Tyr Val Ile Ala Thr Ala Pro Asn Met Phe Asn
                85                  90                  95

Val Asn Asp Val Leu Gly Ala Tyr Ser Pro His Pro Asp Glu Gln Glu
            100                 105                 110

Val Ser Ala Leu Gly Gly Ile Pro Tyr Ser Gln Ile Tyr Gly Trp Tyr
        115                 120                 125

Arg Val His Phe Gly Val Leu Asp Glu Gln Leu His Arg Asn Arg Gly
    130                 135                 140

Tyr Arg Asp Arg Tyr Tyr Ser Asn Leu Asp Ile Ala Pro Ala Ala Asp
145                 150                 155                 160

Gly Tyr Gly Leu Ala Gly Phe Pro Pro Glu His Arg Ala Trp Arg Glu
                165                 170                 175

Glu Pro Trp Ile His His Ala Pro Pro Gly Cys Gly Asn Ala Pro Arg
            180                 185                 190

Ser Ser Met Ser Asn Thr Cys Asp Glu Lys Thr Gln Ser Leu Gly Val
        195                 200                 205

Lys Phe Leu Asp Glu Tyr Gln Ser Lys Val Lys Arg Gln Ile Phe Ser
    210                 215                 220

Gly Tyr Gln Ser Asp Ile Asp Thr His Asn Arg Ile Lys Asp Glu Leu
225                 230                 235                 240

<210>  2
<211>  103
<212>  PRT
<213>  Vibrio cholerae

<400>  2

Thr Pro Gln Asn Ile Thr Asp Leu Cys Ala Glu Tyr His Asn Thr Gln
1               5                   10                  15

Ile His Thr Leu Asn Asp Lys Ile Phe Ser Tyr Thr Glu Ser Leu Arg
            20                  25                  30

Gly Lys Arg Glu Met Ala Ile Ile Thr Phe Lys Asn Gly Ala Thr Phe
        35                  40                  45

Gln Val Glu Val Pro Gly Ser Gln His Ile Asp Ser Gln Lys Lys Ala
    50                  55                  60

Ile Glu Arg Met Lys Asp Thr Leu Arg Ile Ala Tyr Leu Thr Glu Ala
65                  70                  75                  80

Lys Val Glu Lys Leu Cys Val Trp Asn Asn Lys Thr Pro His Ala Ile
                85                  90                  95

Ala Ala Ile Ser Met Ala Asn
            100

<210>  3
<211>  240
<212>  PRT
<213>  Escherichia coli

<400>  3

Asn Gly Asp Lys Leu Tyr Arg Ala Asp Ser Arg Pro Pro Asp Glu Ile
1               5                   10                  15

Lys Arg Ser Gly Gly Leu Met Pro Arg Gly His Asn Glu Tyr Phe Asp

20                  25                  30

Arg Gly Thr Gln Met Asn Ile Asn Leu Tyr Asp His Ala Arg Gly Thr
        35                  40                  45

Gln Thr Gly Phe Val Arg Tyr Asp Asp Gly Tyr Val Ser Thr Ser Leu
    50                  55                  60

Ser Leu Arg Ser Ala His Leu Ala Gly Gln Ser Ile Leu Ser Gly Tyr
65                  70                  75                  80

Ser Thr Tyr Tyr Ile Tyr Val Ile Ala Thr Ala Pro Asn Met Phe Asn
                85                  90                  95

Val Asn Asp Val Leu Gly Val Tyr Ser Pro His Pro Tyr Glu Gln Glu
            100                 105                 110

Val Ser Ala Leu Gly Gly Ile Pro Tyr Ser Gln Ile Tyr Gly Trp Tyr
        115                 120                 125

Arg Val Asn Phe Gly Val Ile Asp Glu Arg Leu His Arg Asn Arg Glu
    130                 135                 140

Tyr Arg Asp Arg Tyr Tyr Arg Asn Leu Asn Ile Ala Pro Ala Glu Asp
145                 150                 155                 160

Gly Tyr Arg Leu Ala Gly Phe Pro Pro Asp His Gln Ala Trp Arg Glu
                165                 170                 175

Glu Pro Trp Ile His His Ala Pro Gln Gly Cys Gly Asp Ser Ser Arg
            180                 185                 190

Thr Ile Thr Gly Asp Thr Cys Asn Glu Glu Thr Gln Asn Leu Ser Thr
        195                 200                 205

Ile Tyr Leu Arg Lys Tyr Gln Ser Lys Val Lys Arg Gln Ile Phe Ser
    210                 215                 220

Asp Tyr Gln Ser Glu Val Asp Ile Tyr Asn Arg Ile Arg Asn Glu Leu
225                 230                 235                 240

<210>  4
<211>  103
<212>  PRT
<213>  Escherichia coli

<400>  4

Ala Pro Gln Ser Ile Thr Glu Leu Cys Ser Glu Tyr Arg Asn Thr Gln

1               5                   10                  15

Ile Tyr Thr Ile Asn Asp Lys Ile Leu Ser Tyr Thr Glu Ser Met Ala
            20                  25                  30

Gly Lys Arg Glu Met Val Ile Ile Thr Phe Lys Ser Gly Ala Thr Phe
        35                  40                  45

Gln Val Glu Val Pro Gly Ser Gln His Ile Asp Ser Gln Lys Lys Ala
    50                  55                  60

Ile Glu Arg Met Lys Asp Thr Leu Arg Ile Thr Tyr Leu Thr Glu Thr
65                  70                  75                  80

Lys Ile Asp Lys Leu Cys Val Trp Asn Asn Lys Thr Pro Asn Ser Ile
                85                  90                  95

Ala Ala Ile Ser Met Glu Asn
            100

<210>  5
<211>  240
<212>  PRT
<213>  Escherichia coli

<400>  5

Asn Gly Asp Lys Leu Tyr Arg Ala Asp Ser Arg Pro Pro Asp Glu Ile
1               5                   10                  15

Lys Arg Ser Gly Gly Leu Met Pro Arg Gly His Asn Glu Tyr Phe Asp
            20                  25                  30

Arg Gly Thr Gln Met Asn Ile Asn Leu Tyr Asp His Ala Arg Gly Thr
        35                  40                  45

Gln Thr Gly Phe Val Arg Tyr Asp Asp Gly Tyr Val Ser Thr Ser Leu
    50                  55                  60

Ser Leu Arg Ser Ala His Leu Ala Gly Gln Ser Ile Leu Ser Gly Tyr
65                  70                  75                  80

Ser Thr Tyr Tyr Ile Tyr Val Ile Ala Thr Ala Pro Asn Met Phe Asn
                85                  90                  95

Val Asn Asp Val Leu Gly Val Tyr Ser Pro His Pro Tyr Glu Gln Glu
            100                 105                 110

Val Ser Ala Leu Gly Gly Ile Pro Tyr Ser Gln Ile Tyr Gly Trp Tyr

115                 120                 125

Arg Val Asn Phe Gly Val Ile Asp Glu Arg Leu His Arg Asn Arg Glu
    130                 135                 140

Tyr Arg Asp Arg Tyr Tyr Arg Asn Leu Asn Ile Ala Pro Ala Glu Asp
145                 150                 155                 160

Gly Tyr Arg Leu Ala Gly Phe Pro Pro Asp His Gln Ala Trp Arg Glu
                165                 170                 175

Glu Pro Trp Ile His His Ala Pro Gln Gly Cys Gly Asp Ser Ser Gly
            180                 185                 190

Thr Ile Thr Gly Asp Thr Cys Asn Glu Glu Thr Gln Asn Leu Ser Thr
        195                 200                 205

Ile Tyr Ala Arg Lys Tyr Gln Ser Lys Val Lys Arg Gln Ile Phe Ser
    210                 215                 220

Asp Tyr Gln Ser Glu Val Asp Ile Tyr Asn Arg Ile Arg Asn Glu Leu
225                 230                 235                 240

<210>  6
<211>  240
<212>  PRT
<213>  Vibrio cholerae

<400>  6

Asn Asp Asp Lys Leu Tyr Arg Ala Asp Ser Arg Pro Pro Asp Glu Ile
1               5                   10                  15

Lys Gln Ser Gly Gly Leu Met Pro Arg Gly Gln Ser Glu Tyr Phe Asp
            20                  25                  30

Arg Gly Thr Gln Met Asn Ile Asn Leu Tyr Asp His Ala Arg Gly Thr
        35                  40                  45

Gln Thr Gly Phe Val Arg His Asp Asp Gly Tyr Val Ser Thr Ser Ile
    50                  55                  60

Ser Leu Arg Ser Ala His Leu Val Gly Gln Thr Ile Leu Ser Gly His
65                  70                  75                  80

Ser Thr Tyr Tyr Ile Tyr Val Ile Ala Thr Ala Pro Asn Met Phe Asn
                85                  90                  95

Val Asn Asp Val Leu Gly Ala Tyr Ser Pro His Pro Asp Glu Gln Glu

            100                 105                 110

Val Ser Ala Leu Gly Gly Ile Pro Tyr Ser Gln Ile Tyr Gly Trp Tyr
        115                 120                 125

Arg Val His Phe Gly Val Leu Asp Glu Gln Leu His Arg Asn Arg Gly
    130                 135                 140

Tyr Arg Asp Arg Tyr Tyr Ser Asn Leu Asp Ile Ala Pro Ala Ala Asp
145                 150                 155                 160

Gly Tyr Gly Leu Ala Gly Phe Pro Pro Glu His Arg Ala Trp Arg Glu
                165                 170                 175

Glu Pro Trp Ile His His Ala Pro Pro Gly Cys Gly Asn Ala Pro Gly
            180                 185                 190

Ser Ser Met Ser Asn Thr Cys Asp Glu Lys Thr Gln Ser Leu Gly Val
        195                 200                 205

Lys Phe Ala Asp Glu Tyr Gln Ser Lys Val Lys Arg Gln Ile Phe Ser
    210                 215                 220

Gly Tyr Gln Ser Asp Ile Asp Thr His Asn Arg Ile Lys Asp Glu Leu
225                 230                 235                 240

<210> 7
<211> 240
<212> PRT
<213> Vibrio cholerae

<400> 7

Asn Asp Asp Lys Leu Tyr Arg Ala Asp Ser Arg Pro Pro Asp Glu Ile
1               5                   10                  15

Lys Gln Ser Gly Gly Leu Met Pro Arg Gly Gln Ser Glu Tyr Phe Asp
            20                  25                  30

Arg Gly Thr Gln Met Asn Ile Asn Leu Tyr Asp His Ala Arg Gly Thr
        35                  40                  45

Gln Thr Gly Phe Val Arg His Asp Asp Gly Tyr Val Ser Thr Ser Ile
    50                  55                  60

Ser Leu Arg Ser Ala His Leu Val Gly Gln Thr Ile Leu Ser Gly His
65                  70                  75                  80

Ser Thr Tyr Tyr Ile Tyr Val Ile Ala Thr Ala Pro Asn Met Phe Asn

85                  90                  95

Val Asn Asp Val Leu Gly Ala Tyr Ser Pro His Pro Asp Glu Gln Glu
            100                 105                 110

Val Ser Ala Leu Gly Gly Ile Pro Tyr Ser Gln Ile Tyr Gly Trp Tyr
        115                 120                 125

Arg Val His Phe Gly Val Leu Asp Glu Gln Leu His Arg Asn Arg Gly
    130                 135                 140

Tyr Arg Asp Arg Tyr Tyr Ser Asn Leu Asp Ile Ala Pro Ala Ala Asp
145                 150                 155                 160

Gly Tyr Gly Leu Ala Gly Phe Pro Pro Glu His Arg Ala Trp Arg Glu
                165                 170                 175

Glu Pro Trp Ile His His Ala Pro Pro Gly Cys Gly Asp Ser Ser Gly
            180                 185                 190

Thr Ile Thr Gly Asp Thr Cys Asp Glu Lys Thr Gln Ser Leu Gly Val
        195                 200                 205

Lys Phe Ala Asp Glu Tyr Gln Ser Lys Val Lys Arg Gln Ile Phe Ser
    210                 215                 220

Gly Tyr Gln Ser Asp Ile Asp Thr His Asn Arg Ile Lys Asp Glu Leu
225                 230                 235                 240

<210>  8
<211>  9
<212>  PRT
<213>  Artificial

<220>
<223>  dm CTA replaced portion

<400>  8

Asn Ala Pro Gly Ser Ser Met Ser Asn
1               5

<210>  9
<211>  9
<212>  PRT
<213>  Artificial

<220>
<223>  Mutated section

<400>  9

Asp Ser Ser Gly Thr Ile Thr Gly Asp
1               5

<210>  10
<211>  38
<212>  DNA
<213>  Artificial

<220>
<223>  Synthetic primer

<400>  10
gggggtctct ctagaatgct gcgggagcaa ggcggctg                                38

<210>  11
<211>  55
<212>  DNA
<213>  Artificial

<220>
<223>  Synthetic primer

<400>  11
ggggggaagc ttataattca tccttaattc tattatgtgt atcaatatca gattg              55

<210>  12
<211>  609
<212>  PRT
<213>  Vibrio cholerae

<400>  12

Met Lys Met Ile Gln Arg Pro Leu Asn Trp Leu Val Leu Ala Gly Ala
1               5                   10                  15

Ala Thr Gly Phe Pro Leu Tyr Ala Ala Gln Met Val Met Ile Asp Asp
            20                  25                  30

Ala Ser Met Val Glu Gln Ala Leu Ala Gln Gln Gln Tyr Ser Met Met
        35                  40                  45

Pro Ala Ala Ser Gly Phe Lys Ala Val Asn Thr Val Gln Leu Pro Asn
    50                  55                  60

Gly Lys Val Lys Val Arg Tyr Gln Gln Met Tyr Asn Gly Val Pro Val
65                  70                  75                  80

Tyr Gly Thr Ala Val Val Ala Thr Glu Ser Ser Lys Gly Ile Ser Gln
                85                  90                  95

Val Tyr Gly Gln Met Ala Gln Gln Leu Glu Ala Asp Leu Ser Thr Val
            100                 105                 110

Thr Pro Asp Ile Glu Ser Gln Gln Ala Ile Ala Leu Ala Val Ser His
        115                 120                 125

Phe Gly Glu Gln His Ala Gly Glu Ser Leu Pro Val Glu Asn Glu Ser
    130                 135                 140

Val Gln Leu Met Val Arg Leu Asp Asp Asn Gln Gln Ala Gln Leu Val
145                 150                 155                 160

Tyr Leu Val Asp Phe Phe Val Ala Ser Glu Thr Pro Ser Arg Pro Phe
                165                 170                 175

Tyr Phe Ile Ser Ala Ala Thr Gly Glu Val Leu Asp Gln Trp Asp Gly
            180                 185                 190

Ile Asn His Ala Gln Ala Thr Gly Thr Gly Pro Gly Gly Asn Gln Lys
        195                 200                 205

Thr Gly Arg Tyr Glu Tyr Gly Ser Asn Gly Leu Pro Gly Phe Thr Ile
    210                 215                 220

Asp Lys Thr Gly Thr Thr Cys Thr Met Asn Asn Ser Ala Val Lys Thr
225                 230                 235                 240

Val Asn Leu Asn Gly Gly Thr Ser Gly Ser Thr Ala Phe Ser Tyr Ala
                245                 250                 255

Cys Asn Asn Ser Thr Asn Tyr Asn Ser Val Lys Thr Val Asn Gly Ala
            260                 265                 270

Tyr Ser Pro Leu Asn Asp Ala His Phe Phe Gly Lys Val Val Phe Asp
        275                 280                 285

Met Tyr Gln Gln Trp Leu Asn Thr Ser Pro Leu Thr Phe Gln Leu Thr
    290                 295                 300

Met Arg Val His Tyr Gly Asn Asn Tyr Glu Asn Ala Phe Trp Asp Gly
305                 310                 315                 320

Arg Ala Met Thr Phe Gly Asp Gly Tyr Thr Arg Phe Tyr Pro Leu Val
                325                 330                 335

Asp Ile Asn Val Ser Ala His Glu Val Ser His Gly Phe Thr Glu Gln
            340                 345                 350

Asn Ser Gly Leu Val Tyr Arg Asp Met Ser Gly Gly Ile Asn Glu Ala
        355                 360                 365

Phe Ser Asp Ile Ala Gly Glu Ala Ala Glu Tyr Phe Met Arg Gly Asn
    370                 375                 380

Val Asp Trp Ile Val Gly Ala Asp Ile Phe Lys Ser Ser Gly Gly Leu
385                 390                 395                 400

Arg Tyr Phe Asp Gln Pro Ser Arg Asp Gly Arg Ser Ile Asp His Ala
                405                 410                 415

Ser Gln Tyr Tyr Ser Gly Ile Asp Val His His Ser Ser Gly Val Phe
            420                 425                 430

Asn Arg Ala Phe Tyr Leu Leu Ala Asn Lys Ser Gly Trp Asn Val Arg
        435                 440                 445

Lys Gly Phe Glu Val Phe Ala Val Ala Asn Gln Leu Tyr Trp Thr Pro
    450                 455                 460

Asn Ser Thr Phe Asp Gln Gly Gly Cys Gly Val Val Lys Ala Ala Gln
465                 470                 475                 480

Asp Leu Asn Tyr Asn Thr Ala Asp Val Val Ala Ala Phe Asn Thr Val
                485                 490                 495

Gly Val Asn Ala Ser Cys Gly Thr Thr Pro Pro Pro Val Gly Lys Val
            500                 505                 510

Leu Glu Lys Gly Lys Pro Phe Thr Gly Leu Ser Gly Ser Arg Gly Gly
        515                 520                 525

Glu Asp Phe Tyr Thr Phe Thr Val Thr Asn Ser Gly Ser Val Val Val
    530                 535                 540

Ser Ile Ser Gly Gly Thr Gly Asp Ala Asp Leu Tyr Val Lys Ala Gly
545                 550                 555                 560

Ser Lys Pro Thr Thr Ser Ser Trp Asp Cys Arg Pro Tyr Arg Ser Gly
                565                 570                 575

Asn Ala Glu Gln Cys Ser Ile Ser Ala Val Val Gly Thr Thr Tyr His
            580                 585                 590

Val Met Leu Arg Gly Tyr Ser Asn Tyr Ser Gly Val Thr Leu Arg Leu
        595                 600                 605

Asp

<210>  13
<211>  62
<212>  DNA
<213>  Artificial

<220>
<223>  Synthetic primer

<400>  13
gcaagtatca cctgtaattg ttcctgatga atccccacaa cccggcggtg catgatgaat        60

cc                                                                      62


<210>  14
<211>  70
<212>  DNA
<213>  Artificial

<220>
<223>  Synthetic primer

<400>  14
gattcatcag gaacaattac aggtgatact tgcgatgaaa aaacccaaag tctaggtgta        60

aaattcgctg                                                              70


<210>  15
<211>  28
<212>  DNA
<213>  Artificial

<220>
<223>  Synthetic

<400>  15
ggcgcccatg gtgaaaacgg gggcgaag                                          28


<210>  16
<211>  34
<212>  DNA
<213>  Artificial

<220>
<223>  Synthetic primer

<400>  16
ggcgcccatg ggcaaatatt atacgcaagg cgac                                   34

## Description

### Technical field

The present invention relates primarily to the field of vaccines, in particular adjuvants used in vaccines, as well as methods for manufacture of adjuvants.

### Background

In the field of vaccine development an important consideration is the use of an appropriate adjuvant. Adjuvants are reagents that enhance the immune response to the target antigens with which they are co-administered. Although considerable effort has been expended on developing novel adjuvants only few have been licensed for use in human vaccines. With the advent of mucosally administered vaccines given by e.g. oral or intranasal routes the scarcity of suitable adjuvants has become even more acute. Early successes with the oral polio and cholera vaccines have not been followed through and only a handful mucosal vaccines for human use have been licensed. Although mucosal delivery of vaccines may be needed against many infections in the gastrointestinal, respiratory and genital tracts and would also be attractive for delivery of many other vaccines in terms of ease of administration, antigens delivered through the mucosae tend to be poorly immunogenic and efficient adjuvants are sorely needed.

Some of the most effective mucosal adjuvants known to date are the enterotoxins produced by *Vibrio cholerae* and enterotoxigenic *Escherichia coli* (CT and LT respectively). These closely related toxins are largely responsible for the severe watery diarrhea that results from infection with these organisms. Thus in their native states they are far too toxic to be considered for use as adjuvants in vaccines. These toxins have an AB5 structure in which the B subunit pentamer (CTB or LTB) is responsible for receptor binding and the A subunit (CTA or LTA) has an enzymatic activity associated with toxicity and paradoxically, with adjuvant activity. Both LTA and CTA have the same enzymatic activity catalyzing the ADP ribosylation of the Gₛ GTP binding protein resulting in constitutive production of cAMP which in turn drives the active secretion of fluid and electrolytes across the epithelium into the lumen of the small intestine.

There have been extensive attempts to reduce the toxicity of these enterotoxins whilst maintaining the adjuvant activity of the molecules. This has been done by mutations in the enzymatically active A subunits that affect either the active site significantly reducing or ablating the enzymatic activity, or by disruption of the proteolytic cleavage ("nicking") between the CTA1 and CTA2 parts of the protein that is necessary for full toxicity. Both approaches have had some degree of success, but recent attention has focused on the proteolytic cleavage-resistant mutants of LT. Although LT with a single mutation (R192G) in the A subunit showed some promise, it still proved to have residual toxicity and a further mutation was introduced that reduced toxicity further but resulted in a molecule (LT(R192G/L211A)) with adjuvant activity that approached that of native LT or CT. This double-mutated molecule (termed dmLT) has been studied intensively and is currently undergoing clinical trials.

In an attempt to make an adjuvant that would be suitable for a novel killed whole cell oral cholera vaccine under development in the inventor's laboratory, the inventors made similar mutations to those in dmLT in the CT molecule. The inventors generated *V. cholerae* strains that produce the resulting molecule but surprisingly found that it was still susceptible to cleavage by bacterial proteases secreted by *Vibrio cholerae,* whereby the resulting molecule re-acquired toxicity.

It is an object of the present invention to provide non-toxic derivatives of CTA that are fully resistant to proteolytic cleavage in vivo and under production conditions but with retained adjuvant activity when associated with CTB or analogous cell-binding molecules.

### Definitions

The term *cholera toxin A-subunit,* abbreviated "CTA" refers to the toxic-active ADP-ribosylating A subunit of cholera toxin. Wild-type CTA has an amino-acid sequence according to SEQ 10 NO: 1.

The term *cholera toxin B-subunit,* abbreviated "CTB" refers to the cell-binding B subunit, present as a pentamer in the native cholera toxin. Wild-type CTB has an amino-acid sequence according to SEQ ID NO: 2.

The term *heat-labile enterotoxin A-subunit,* abbreviated "LTA" refers to the toxic-active ADP-ribosylating A subunit of E. coli heat-labile enterotoxin. LTA is highly homologous to CTA. Wild-type LTA has an amino-acid sequence according to SEQ ID NO: 3.

The term *heat-labile enterotoxin B-subunit,* abbreviated "LTB" refers to the cell-binding B subunit present as a pentamer in the *heat-labile enterotoxin.* LTB is highly homologous to CTB. Wild-type CTB has an amino-acid sequence according to SEQ ID NO: 4.

The term *GM1 ganglioside* refers to monosialoganglioside GM1 with the structure {Gal(β1-3)GalNac(β1-4)(NeuA-c(α2-3)Gal(β1-4)Glc}-ceramide, the primary cellular receptor to which CTB is known to bind.

A given *percentage sequence identity* in the context of the present invention refers to sequence identify as calculated by the BLAST-algorithm (Altschul, S.F., Gish, W., Miller, W., Myers, E.W. & Lipman, D.J. (1990) "Basic local alignment search tool." J. Mol. Biol. 215:403-410) for a pairwise sequence alignment made using the BLAST algorithm. A preferred online implementation of the BLAST algorithm is found at http://blast.ncbi.nlm.nih.gov/Blast.cgi.

*Vibrio cholerae* VesA- protease refers to the gene product of the vesA gene of Vibrio cholerae and is a serine protease.

*Vibrio cholerae* HA- protease (HAP) also referred to as soluble hemagglutinin is a metalloenzyme non-serine protease which is inhibited by chelating agents such as EGTA and inhibitors of zinc metalloproteases. It is the processed product of the hapA gene which encodes the polypeptide of SEQ ID NO: 12, obtained from the complete genome of chromosome II of *V.cholerae* 0395 GenBank accession number NC_012583.1.

The term *trypsin* refers to a serine protease that is found in the digestive tract of many vertebrates and which cleaves peptides on the carboxyl side of amino acid residues lysine or arginine except where such residues are followed by proline.

Single-letter designations of amino-acids have their commonly understood meaning in the art.

All references disclosed herein are incorporated by reference.

### Brief description of the figures

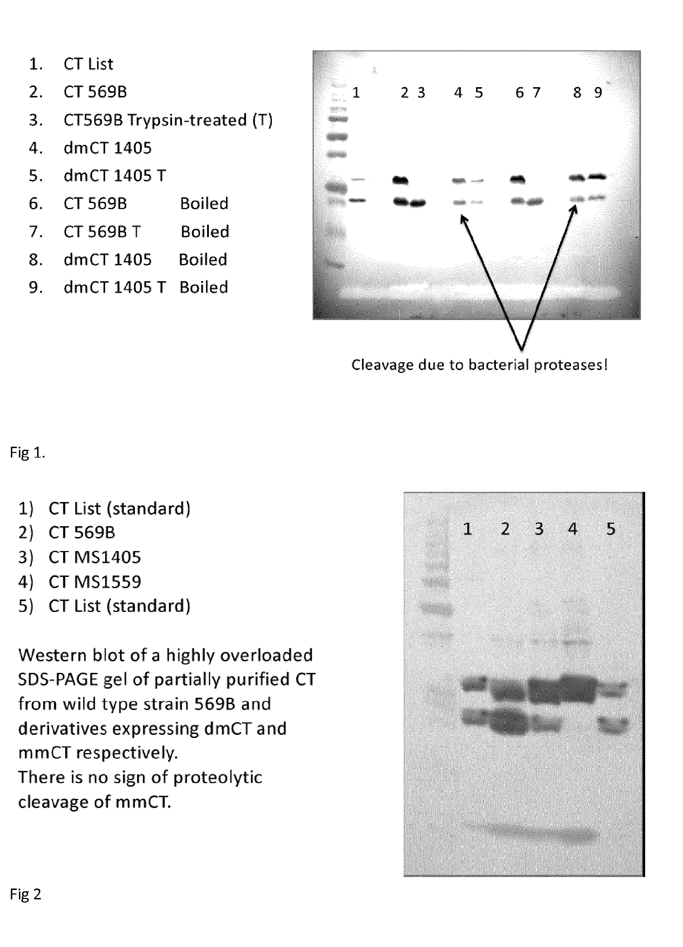

**Fig 1****: While dmCT is resistant to trypsin and more resistant to *V. cholerae* proteases than native CT, it is still partly "nicked" in the A subunit by the bacterial proteases.**
**Fig 2****: mmCT is resistant to bacterial proteases.** Western blot of a highly overloaded SDS-PAGE gel of partially purified CT from wild type strain 569B and derivatives expressing dmCT and mmCT respectively. There is no sign of proteolytic cleavage of mmCT.

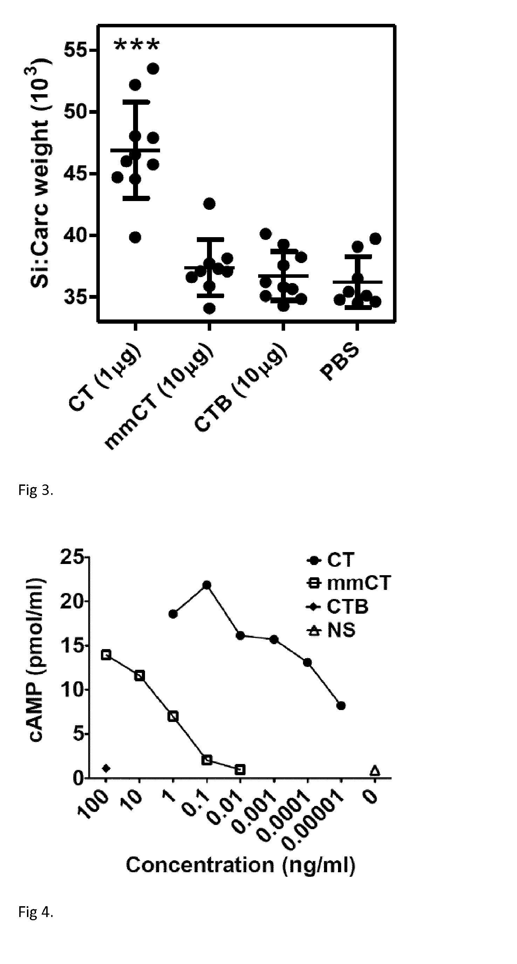

**Fig 3****: mmCT is non-toxic in the infant mouse enterotoxicity assay.** Three day old mice (C57/BL6) were intragastrically (i.g.) administered either 1 microgram CT (n = 10), 10 microgram mmCT (n = 9), 10 microgram CTB (n = 10) or buffer (PBS) (n = 8). After 18h the mice were weighed individually and following sacrifice intestines were examined for fluid accumulation by weighing the intestines and the remaining carcass and calculating the ratio as a measure of relative intestinal fluid accumulation. The mmCT is completely non-toxic causing no fluid accumulation in contrast to CT which induces strong intestinal fluid secretion. *** p<0.001 for difference between the CT and mmCT groups.
**Fig 4****: Induction of cyclic AMP production in mouse thymocytes by CT and mmCT.** Single cell suspension of mouse thymocytes (5x10⁶) were treated with various concentrations of CT, mmCT, CTB, or left untreated. After 2.5h incubation at 37° C the cells were lysed and cAMP P production was analysed by ELISA. mmCT was ca 100,000 fold less potent than native CT.

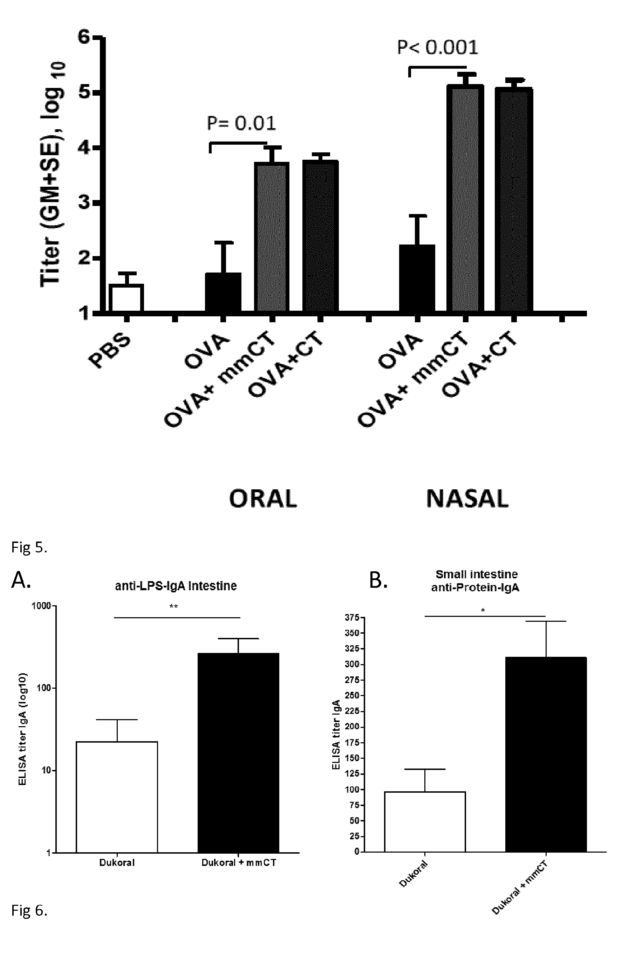

**Fig 5****: Strong adjuvant activity of mmCT on serum antibody response to a mucosally co-administered model protein antigen.** Serum IgG antibody response to ovalbumin (OVA) were quantified after oral/i.g. or nasal immunization. Sera collected 2 weeks after the last of 2 immunisations with OVA (1 mg orally or 0.2 mg nasally) given alone or together with mmCT or CT (10 µg orally or 2 µg nasally) were tested for IgG anti-OVA antibodies by ELISA and log-transformed titers are shown as geometric means + SEM.
**Fig 6****: Strong adjuvant activity of mmCT on intestinal-mucosal IgA antibody production.** Responses to V. cholerae lipopolysaccharide (LPS) (A) and protein antigen (B) in mice after intragastric immunization with the Dukoral^{®} oral cholera vaccine are shown. Mice were mucosally immunized by the oral/intragastric route as described in Methods and after two rounds of immunization the mice were sacrificed and small intestinal extracts prepared and tested by ELISA for locally produced specific IgA antibodies against V. cholerae 01 LPS (A) and protein antigen (B). Results show significantly increased antibody levels in mice that received the oral cholera vaccine together with mmCT.

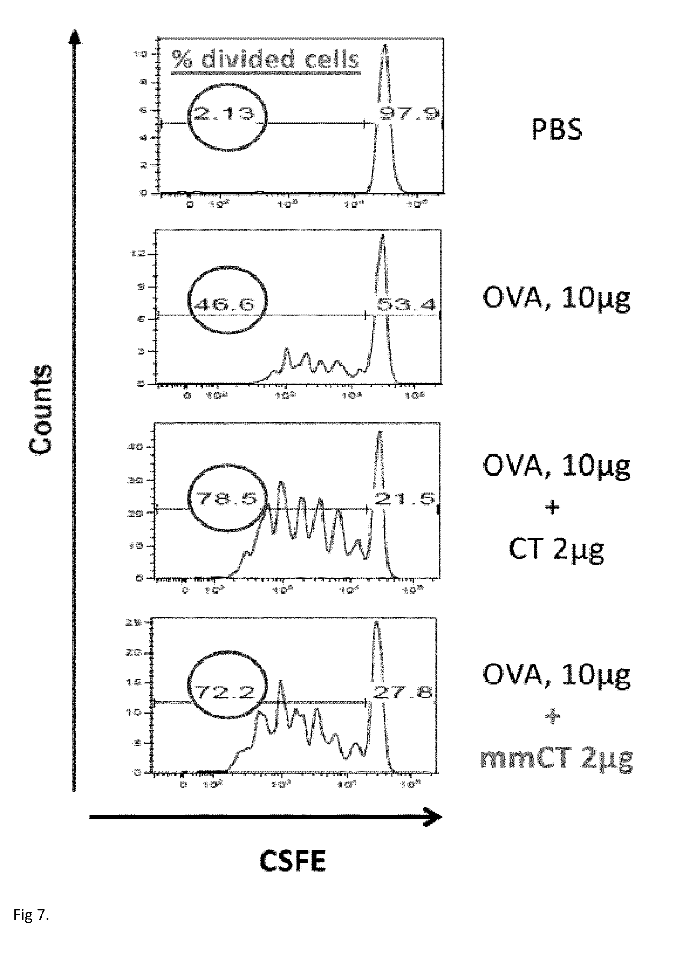

**Fig 7****: Adjuvant effect of mmCT on OVA-specific (OT-II) CD4+ T cell division in cervical lymph nodes in response to nasally co-administered OVA antigen.** Mice were adoptively transferred with OVA-specific OT-II CD4⁺ T cells labelled ex vivo with CFSE; one day later the mice were immunized intranasally (I.N.) with a single dose of PBS, OVA, OVA + CT, or OVA + mmCT in the doses shown. Three days later the mice were sacrificed and cervical lymph node CD4⁺ lymphocytes isolated and examined for cell division by analysing their extent of CFSE staining by flow cytometry. Mice receiving OVA together with mmCT exhibit stronger cell division than mice receiving only OVA and similar extent of cell division as mice receiving CT.

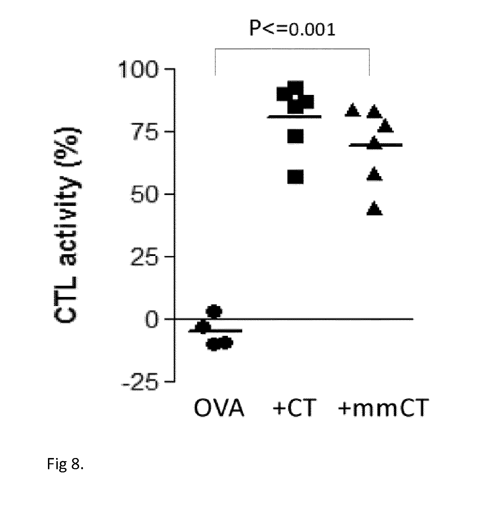

**Fig 8****: Strong adjuvant activity of mmCT on mucosal induction of antigen-specific CD8+ cytotoxic lymphocytes (CTLs).** C57BL/6 mice were immunized intranasally (I.N.) with PBS, OVA (5 mg), or the same dose of OVA supplemented with CT or mmCT (1.5mg for both toxins). They were then injected with 1:1 mixture of splenocytes either pulsed or not pulsed with the MHC class I restricted OT1 peptide from OVA. The two groups were labelled with different amounts of CFSE. Results are expressed as % specific lysis as detemined by loss of the OT1 pulsed splenocytes compared to the non-pulsed controls and show strong CTL activity in mice immunized with OVA+mmCT being similar to that in mice immunized with OVA+CT and much higher than the undetectable CTL activity in mice immunized with OVA alone..

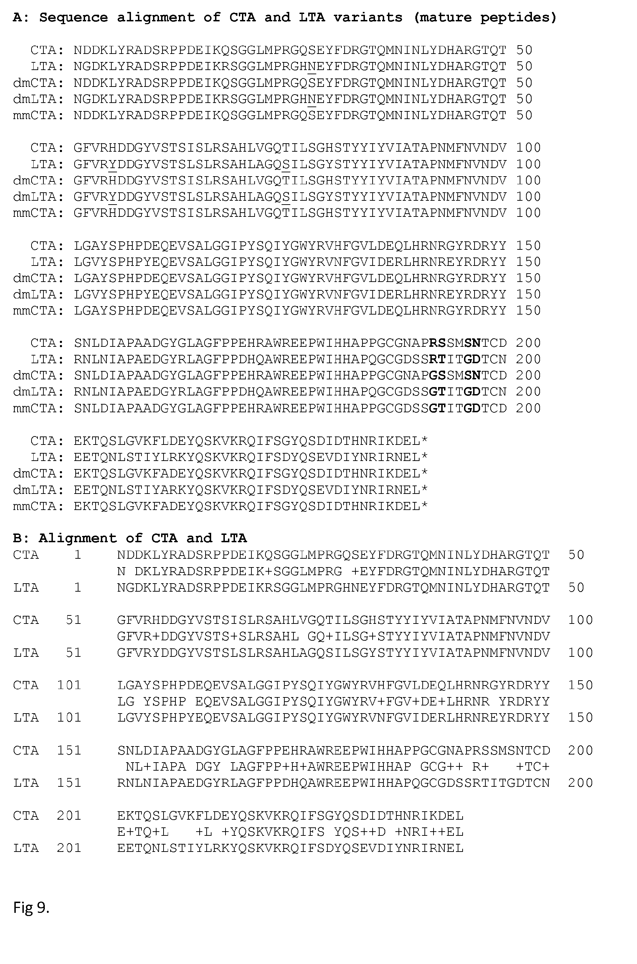

**Fig 9****: A) Sequence alignment of mature CTA and LTA variants relevant to the present invention. B) Sequence alignment of wild-type CTA and LTA.** High degree of homology can be observed. The trypsing and HAP cleavage sites between positions 192/193 and 197/198, respectively are illustrated in bold.

### Summary of the invention

The present invention relates to and provides the following items, whose contents are to be interpreted as if they were patent claims. However, the scope of patent protection sought will ultimately be determined solely by the claims.
1. A cholera toxin A subunit (CTA)-like polypeptide having at least 90% sequence identity to CTA (SEQ ID NO: 1), **characterized in that**:
   a. the CTA-like polypeptide contains one or more mutations in its sequence rendering the trypsin cleavage site between amino-acids 192 and 193 of CTA trypsin-resistant; and
   b. the CTA-like polypeptide contains one or more mutations in its sequence rendering the *Vibrio cholerae* HAP cleavage site between amino-acids 197 and 198 of CTA HAP-resistant.
2. The polypeptide according to item 1, wherein trypsin-resistance and HAP-resistance are defined such that the trypsin and HAP cleavage sites are cleaved at least 10-fold slower by trypsin and HAP, respectively, compared to corresponding sites in wild-type CTA (SEQ ID NO: 1) under corresponding conditions.
3. The polypeptide according to item 2, wherein said protease cleavage sites are cleaved at least 100-fold slower.
4. The polypeptide according to item 3 wherein said protease cleavage sites are cleaved at least 1000-fold slower.
5. The polypeptide according to any of the preceding items, wherein the polypeptide comprises one or more mutations in amino-acid residues aligning with residues 189-200 of SEQ ID NO:1, compared to SEQ ID NO: 1.
6. The polypeptide according to any of the preceding items, wherein the polypeptide comprises at least 2 mutations in amino-acid residues aligning with residues 189-200 of SEQ ID NO:1.
7. The polypeptide according to any of the preceding items, wherein the polypeptide comprises at least 3 mutations in amino-acid residues aligning with residues 189-200 of SEQ ID NO:1.
8. The polypeptide according to any of the preceding items, wherein the polypeptide comprises at least 4 mutations in amino-acid residues aligning with residues 189-200 of SEQ ID NO:1.
9. The polypeptide according to any of the preceding items, wherein the polypeptide comprises an amino acid sequence having at least 95 % identity to SEQ ID NO: 1.
10. The polypeptide according to any of the preceding items, wherein the polypeptide comprises an amino acid sequence having at least 98 % identity to SEQ ID NO: 1.
11. The polypeptide according to any of the preceding items, wherein the polypeptide comprises an amino acid sequence having at least 99 % identity to SEQ ID NO: 1.
12. The polypeptide according to any of the preceding items, wherein the polypeptide contains a mutation of the residue aligning with residue N189 of SEQ ID NO:1.
13. The polypeptide according to any of the preceding items, wherein the polypeptide contains a mutation of the residue aligning with residue A190 of SEQ ID NO:1.
14. The polypeptide according to any of the preceding items, wherein the polypeptide contains a mutation of the residue aligning with residue P191 of SEQ ID NO:1.
15. The polypeptide according to any of the preceding items, wherein the polypeptide contains a mutation of the residue aligning with residue R192 of SEQ ID NO:1.
16. The polypeptide according to any of the preceding items, wherein the polypeptide contains a mutation of the residue aligning with residue S193 of SEQ ID NO:1.
17. The polypeptide according to any of the preceding items, wherein the polypeptide contains a mutation of the residue aligning with residue S194 of SEQ ID NO:1.
18. The polypeptide according to any of the preceding items, wherein the polypeptide contains a mutation of the residue aligning with residue M195 of SEQ ID NO:1.
19. The polypeptide according to any of the preceding items, wherein the polypeptide contains a mutation of the residue aligning with residue S196 of SEQ ID NO:1.
20. The polypeptide according to any of the preceding items, wherein the polypeptide contains a mutation of the residue aligning with residue N197 of SEQ ID NO:1.
21. The polypeptide according to any of the preceding items, wherein the polypeptide contains a mutation of the residue aligning with residue T198 of SEQ ID NO:1.
22. The polypeptide according to any of the preceding items, wherein the polypeptide contains a mutation of the residue aligning with residue C199 of SEQ ID NO:1.
23. The polypeptide according to any of the preceding items, wherein the polypeptide contains a mutation of the residue aligning with residue D200 of SEQ ID NO:1.
24. The polypeptide according to any of the preceding items, wherein the polypeptide contains a mutation of the residue aligning with residue L211 of SEQ ID NO:1.
25. The polypeptide according to any of the preceding items, wherein the polypeptide contains a mutation of the residue aligning with residue N189 of SEQ ID NO:1 to D.
26. The polypeptide according to any of the preceding items, wherein the polypeptide contains a mutation of the residue aligning with residue A190 of SEQ ID NO:1 to S.
27. The polypeptide according to any of the preceding items, wherein the polypeptide contains a mutation of the residue aligning with residue P191 of SEQ ID NO:1 to S
28. The polypeptide according to any of the preceding items, wherein the polypeptide contains a mutation of the residue aligning with residue R192 of SEQ ID NO:1 to G.
29. The polypeptide according to any of the preceding items, wherein the polypeptide contains a mutation of the residue aligning with residue S193 of SEQ ID NO:1 to T.
30. The polypeptide according to any of the preceding items, wherein the polypeptide contains a mutation of the residue aligning with residue S194 of SEQ ID NO:1 to I.
31. The polypeptide according to any of the preceding items, wherein the polypeptide contains a mutation of the residue aligning with residue M195 of SEQ ID NO:1 to T.
32. The polypeptide according to any of the preceding items, wherein the polypeptide contains a mutation of the residue aligning with residue S196 of SEQ ID NO:1 to G.
33. The polypeptide according to any of the preceding items, wherein the polypeptide contains a mutation of the residue aligning with residue N197 of SEQ ID NO:1 to D.
34. The polypeptide according to any of the preceding items, wherein the polypeptide contains a mutation of the residue aligning with residue L211 of SEQ ID NO:1 to A.
35. The polypeptide according to any of the preceding items, wherein the polypeptide contains the sequence DSSGTITGD (SEQ ID NO: 2) in place of the residues aligning with residues 189-197 of SEQ ID NO:1.
36. The polypeptide according to any of the preceding items, wherein the polypeptide has the sequence according to SEQ ID NO: 7.
37. A polynucleotide encoding a CTA-like polypeptide according to any of the preceding items.
38. A vector comprising a polynucleotide according to item 37.
39. A host cell comprising a polynucleotide according to item 37 or a vector according to item 38.
40. The host cell of item 39, wherein the host cell is a bacterial cell.
41. The host cell of item 40, wherein the host cell is a *Vibrio cholerae* cell.
42. The host cell according to any of items 39-41, wherein the polynucleotide is expressed by the host cell.
43. A method for producing a CTA-like polypeptide according to any of items 1-36, comprising the steps of:
   a. providing a host cell according to item 42;
   b. culturing said host cell in conditions such that the polypeptide is produced by the host cell; and
   c. recovering said produced polypeptide unit from the culture.
44. An adjuvant with low toxicity, comprising
   a. a cholera toxin A subunit (CTA)-like polypeptide according to any of items 1-36,associated with
   b. a unit promoting cellular entry of said CTA-like polypeptide into antigen-presenting cells.
45. The adjuvant according to item 44, wherein the unit promoting cellular entry is a protein A derivative termed DD.
46. The adjuvant according to item 44, wherein the unit promoting cellular entry is a GM₁ ganglioside-binding polypeptide unit.
47. The adjuvant according to item 46 wherein the GM₁-ganglioside binding polypeptide unit is a polypeptide which is immunologically cross-reactive with antibodies raised against CTB (SEQ ID NO: 2) or LTB (SEQ ID NO: 4).
48. The adjuvant according to any of items 46-47 wherein the GM₁-ganglioside binding polypeptide unit has at least 90% sequence identity to CTB (SEQ ID NO: 2) or LTB (SEQ ID NO: 4).
49. The adjuvant according to item 48, wherein the GM₁-ganglioside binding polypeptide unit is CTB (SEQ ID NO: 2).
50. The adjuvant according to any of items 44-49, wherein the adjuvant possesses low toxicity on mucosal administration to a mammal, wherein said low toxicity amounts to at least 10-fold lower toxicity compared to wild-type cholera toxin on a molar basis.
51. The adjuvant according to item 50, wherein the adjuvant possesses low toxicity on mucosal administration to a mammal, wherein said low toxicity amounts to at least 100-fold lower toxicity compared to wild-type cholera toxin on a molar basis.
52. The adjuvant according to any of items 44-51, wherein the toxicity is defined by means of analysing cyclic AMP production in mammalian cells or tissues, whereby the cyclic AMP production is at least 10-fold lower than of wild-type cholera toxin on a molar basis.
53. The adjuvant according to item 52, wherein the toxicity is defined by means of analysing cyclic AMP production in mammalian cells or tissues, whereby the cyclic AMP production is at least 100-fold lower than of wild-type cholera toxin on a molar basis.
54. The adjuvant according to any of items 52-53, wherein the cyclic AMP production is measured *in vitro* in mouse thymocytes.
55. The adjuvant according to any of items 44-54 wherein on oral administration to a mammal, said effective adjuvant activity amounts to at least 10% adjuvant activity compared to an equimolar dose of wild-type cholera toxin.
56. The adjuvant according to item 55, wherein the effective adjuvant activity amounts to at least 30% of that of an equimolar dose of wild-type cholera toxin.
57. The adjuvant according to any of items 44-56, wherein the effective adjuvant activity is measured in terms of quantitative antibody formation in response to oral *in vivo* administration of a suitable antigen together with the adjuvant.
58. The adjuvant according to any of items 44-57 for use in therapy or prophylaxis.
59. The adjuvant according to any of items 44-58 for use as an adjuvant in protective immunization.
60. A composition comprising a CTA-like polypeptide according to any of items 1-36 and a pharmaceutically acceptable excipient, carrier or diluent.
61. The composition according to item 60, wherein the composition is a vaccine.
62. The composition according to any of items 60-61 wherein the composition further comprises an antigen or an antigen epitope.
63. A vaccine comprising an adjuvant according to any of items 44-57, or a composition according to item 62.
64. The vaccine according to item 63, further comprising an antigen from an enterotoxigenic *E. coli* or a *Vibrio cholerae.*
65. The vaccine according to any of items 63-64, for use in mucosal administration.
66. The vaccine for use according to item 65, wherein the mucosal administration is nasal or oral administration.
67. The vaccine for use according to item 65, wherein the mucosal administration is oral, sublingual, intragastric or rectal administration, administration to a respiratory mucosa such as by intranasal or pulmonary administration, administration to a genital mucosa such as by cervical or vaginal application, or administration to the eye mucosa such as by eye drops.
68. The vaccine according to any of items 63-64 or the vaccine for use according to any of items 65-67, for use in eliciting protective immunity against a pathogen.
69. The vaccine for use according to item 68, wherein the pathogen is enterotoxigenic *E. coli* or *Vibrio cholerae disease.*
70. A use of a polypeptide according to any of items 1-36 in a vaccine.
71. A method for production of a reduced toxicity CTA- like polypeptide in a *Vibrio cholerae*-host, comprising the steps of:
   a. providing a polynucleotide encoding for a polypeptide having at least 70 % sequence identity to cholera toxin A subunit (CTA, SEQ ID NO: 1), said polypeptide containing one or more mutations in its sequence rendering the trypsin cleavage site between amino-acids 192 and 193 of CTA trypsin-resistant and the HAP cleavage site between amino-acids 197 and 198 of CTA HAP-resistant;
   b. introducing said polynucleotide to a suitable *Vibrio cholerae* host cell such that the polypeptide is expressed by the host cell;
   c. culturing said host cell in conditions such that the polypeptide is produced by the host cells; and
   d. recovering said produced polypeptide from the culture; wherein the produced CTA-like polypeptide has reduced toxicity due to resistance to proteolytic activation.
72. The method according to item 71, wherein the encoded protein has at least 80% sequence identity to wild-type CTA (SEQ ID NO: 1).
73. The method according to any of items item 71-72, wherein trypsin-resistance and HAP-resistance are defined such that the trypsin and HAP cleavage sites are cleaved at least 10-fold, preferably 100-fold, most preferably 1000-fold slower by trypsin and HAP, respectively, compared to corresponding sites in wild-type CTA under corresponding conditions.
74. The method according to any of items 71-73, wherein the host cells concomitantly express a polypeptide capable of associating with the CTA-like polypeptide and promoting cellular entry of the CTA-like polypeptide into antigen-presenting cells.
75. The method according to any of items 71-74, wherein the host cells concomitantly express a GM₁-ganglioside binding polypeptide capable of associating with said produced polypeptide.
76. The method according to item 75, wherein the GM₁-ganglioside binding polypeptide is a polypeptide which is immunologically cross-reactive with antibodies raised against CTB (SEQ ID NO: 2) or LTB (SEQ ID NO: 4).
77. The method according to any of items 75-76, wherein the GM₁-ganglioside binding polypeptide unit has at least 90% sequence identity to CTB (SEQ ID NO: 2) or LTB (SEQ ID NO: 4).
78. The method according to item 77, wherein the GM₁-ganglioside binding polypeptide unit is CTB.
79. The method according to any of items 71-78, wherein the CTA-like polypeptide comprises one or more mutations in amino-acid residues aligning with residues 189-200 of SEQ ID NO:1.
80. The method according to any of items 71-79, wherein the CTA-like polypeptide comprises at least 2 mutations in amino-acid residues aligning with residues 189-200 of SEQ ID NO:1.
81. The method according to any of items 71-80, wherein the CTA-like polypeptide comprises at least 3 mutations in amino-acid residues aligning with residues 189-200 of SEQ ID NO:1.
82. The method according to any of items 71-81, wherein the CTA-like polypeptide comprises at least 4 mutations in amino-acid residues aligning with residues 189-200 of SEQ ID NO:1.
83. The method according to any of items 71-82, wherein the CTA-like polypeptide comprises an amino acid sequence having at least 95 % identity to SEQ ID NO: 1.
84. The method according to any of items 71-83, wherein the CTA-like polypeptide comprises an amino acid sequence having at least 98 % identity to SEQ ID NO: 1.
85. The method according to any of items 71-84, wherein the CTA-like polypeptide comprises an amino acid sequence having at least 99 % identity to SEQ ID NO: 1.
86. The method according to any of items 71-85, wherein the CTA-like polypeptide contains a mutation of the residue aligning with residue N189 of SEQ ID NO:1.
87. The method according to any of items 71-86, wherein the CTA-like polypeptide contains a mutation of the residue aligning with residue A190 of SEQ ID NO:1.
88. The method according to any of items 71-87, wherein the CTA-like polypeptide contains a mutation of the residue aligning with residue P191 of SEQ ID NO:1.
89. The method according to any of items 71-88, wherein the CTA-like polypeptide contains a mutation of the residue aligning with residue R192 of SEQ ID NO:1.
90. The method according to any of items 71-89, wherein the CTA-like polypeptide contains a mutation of the residue aligning with residue S193 of SEQ ID NO:1.
91. The method according to any of items 71-90, wherein the CTA-like polypeptide contains a mutation of the residue aligning with residue S194 of SEQ ID NO:1.
92. The method according to any of items 71-91, wherein the CTA-like polypeptide contains a mutation of the residue aligning with residue M195 of SEQ ID NO:1.
93. The method according to any of items 71-92, wherein the CTA-like polypeptide contains a mutation of the residue aligning with residue S196 of SEQ ID NO:1.
94. The method according to any of items 71-93, wherein the CTA-like polypeptide contains a mutation of the residue aligning with residue N197 of SEQ ID NO:1.
95. The method according to any of items 71-94, wherein the CTA-like polypeptide contains a mutation of the residue aligning with residue T198 of SEQ ID NO:1.
96. The method according to any of items 71-95, wherein the CTA-like polypeptide contains a mutation of the residue aligning with residue C199 of SEQ ID NO:1.
97. The method according to any of items 71-96, wherein the CTA-like polypeptide contains a mutation of the residue aligning with residue D200 of SEQ ID NO:1.
98. The method according to any of items 71-97, wherein the CTA-like polypeptide contains a mutation of the residue aligning with residue L211 of SEQ ID NO:1.
99. The method according to any of items 71-98, wherein the CTA-like polypeptide contains a mutation of the residue aligning with residue N189 of SEQ ID NO:1 to D.
100. The method according to any of items 71-99, wherein the CTA-like polypeptide contains a mutation of the residue aligning with residue A190 of SEQ ID NO:1 to S.
101. The method according to any of items 71-100, wherein the CTA-like polypeptide contains a mutation of the residue aligning with residue P191 of SEQ ID NO:1 to S
102. The method according to any of items 71-101, wherein the CTA-like polypeptide contains a mutation of the residue aligning with residue R192 of SEQ ID NO:1 to G.
103. The method according to any of items 71-102, wherein the CTA-like polypeptide contains a mutation of the residue aligning with residue S193 of SEQ ID NO:1 to T.
104. The method according to any of items 71-103, wherein the CTA-like polypeptide contains a mutation of the residue aligning with residue S194 of SEQ ID NO:1 to I.
105. The method according to any of items 71-104, wherein the CTA-like polypeptide contains a mutation of the residue aligning with residue M195 of SEQ ID NO:1 to T.
106. The method according to any of items 71-105, wherein the CTA-like polypeptide contains a mutation of the residue aligning with residue S196 of SEQ ID NO:1 to G.
107. The method according to any of items 71-106, wherein the CTA-like polypeptide contains a mutation of the residue aligning with residue N197 of SEQ ID NO:1 to D.
108. The method according to any of items 71-107, wherein the CTA-like polypeptide contains a mutation of the residue aligning with residue L211 of SEQ ID NO:1 to A.
109. The method according to any of items 71-108, wherein the CTA-like polypeptide contains the sequence DSSGTITGD (SEQ ID NO: 9) in place of the residues aligning with residues 189-197 of SEQ ID NO:1 (SEQ ID NO: 8).
110. The method according to any of items 71-109, wherein the CTA-like polypeptide has the sequence according to SEQ ID NO: 7.

### Detailed description

### CTA-like polypeptide

In a first aspect, the present invention provides a cholera toxin A subunit (CTA)-like polypeptide having at least 90% sequence identity to CTA (SEQ ID NO: 1), **characterized in that**:
a. the CTA-like polypeptide contains one or more mutations in its sequence rendering the trypsin cleavage site between amino-acids 192 and 193 of CTA trypsin-resistant; and
b. the CTA-like polypeptide contains one or more mutations in its sequence rendering the *Vibrio cholerae* HAP cleavage site between amino-acids 197 and 198 of CTA HAP-resistant.

The trypsin resistance has the effect that the CTA-like polypeptide cannot be processed into active form by host trypsin after *in vivo* administration. The HAP-resistance has the effect that the CTA-like polypeptide cannot be processed into active form by *Vibrio cholerae* HAP protease, which is particularly relevant for production of a CTA-like polypeptide in *in vitro* cultures. In effect, combining trypsin resistance with resistance to HAP cleavage enables the production of the CTA-like protein *in vitro* using *V. cholerae* as a host.

It is to be understood that while HAP is a major bacterial protease relevant in the production of CTA derivatives in a *V. cholerae* host, it is likely not the only protease that may nick CTA. Another protease being a candidate for nicking CTA is a serine protease VesA, which nicks the CTA produced by cells lacking HAP activity (Sikora et al. J Biol Chem. 2011 May 13; 286(19): 16555-16566). Notwithstanding the potential that the CTA molecule might be processed by several proteases (including yet to be identified proteases), for the practical purposes of providing an advantageous novel CTA-derivative (see more below), it is sufficient that the molecule is rendered resistant to HAP.

Since proteolytic cleavage (so-called "nicking") of CTA is a prerequisite for its intracellular toxic activity, the inactivation of the susceptibility to both mammalian host and bacterial proteases renders the CTA-like polypeptide practically non-toxic for mammalian cells or whole animals. The ability to produce this non-toxic molecule in *V. cholerae* is a major advantage since it can then readily be isolated with high yield from the extracellular medium alone or in association with CTB or related secreted cell-binding proteins.

In summary, major advantages of the novel CTA-like polypeptide include but are not limited to:
- It can be produced in *Vibrio cholerae* which secretes the product almost quantitatively into the growth medium.
- Production in *Vibrio cholerae* has the advantage that the polypeptide is readily produced in large quantities, compared to e.g. *E. coli* as a host.
- Production in *Vibrio cholerae* has the advantage that the polypeptide can easily be purified from the medium of a *Vibrio cholerae* culture, compared to using e.g. *E. coli* host where CTA ends up in periplasmic space where several other proteins contaminate the product.
- The polypeptide can be produced in a strain of *Vibrio cholerae* the cells of which can be potentially used as a vaccine in their own right, facilitating cost-effective production of a vaccine for cholera. It is to be noted that for a cholera vaccine, that is to be successful in broad use in low-income countries, a low price per unit dose is a crucial factor.
- The polypeptide can be produced in a strain of *Vibrio cholerae* that does not have additional mutations (for example in hapA or vesA) that would be required to stabilize more sensitive derivatives of CT (such as dmCT) but would also compromise the growth of the organisms in culture and the production capacity.
- The novel CTA polypeptide has significantly lower toxicity than the native toxin, yet it retains a broad range of biological properties that contribute to excellent adjuvanticity.

The trypsin-resistance and HAP-resistance may be defined such that the trypsin and HAP cleavage sites are cleaved at least 10-fold slower by trypsin and HAP, respectively, compared to corresponding sites in wild-type CTA (SEQ ID NO: 1) under corresponding conditions. Preferably, said protease cleavage sites are cleaved at least 100-fold slower. More preferably, said protease cleavage sites are cleaved at least 1000-fold slower. The conditions under which the difference in cleavage speed is measured are preferably similar to culture conditions for *V. cholerae.*

In the Classical V. cholerae strains used in the Examples herein to express the mutant mmCT optimal expression of the CT occurs at 30° C. When dmCT is grown at this temperature there is significantly more cleavage presumably resulting in toxicity since this molecule is still sensitive to bacterial proteases despite being resistant to cleavage by trypsin. Thus conditions that optimize expression of CT also optimize expression of enzymes that will cleave the CTA molecule. The inventors have therefore compared the cleavage of the different CT derivatives under conditions where one would expect cleavage of the native CT to be maximal.

The protease resistance of the polypeptide of the first aspect may be due to one, two, three, four, five, six, seven, eight, nine, ten, eleven or twelve mutations in amino-acid residues aligning with residues 189-200 of SEQ ID NO:1, compared to SEQ ID NO: 1.

The polypeptide of the first aspect may comprise an amino acid sequence having at least 91%, 92%, 93%, 94%, 95%, 96% or 97% identity to SEQ ID NO: 1, preferably at least 98 % identity to SEQ ID NO: 1, more preferably at least 99 % identity to SEQ ID NO: 1.

The polypeptide of the first aspect may contain the sequence DSSGTITGD (SEQ ID NO: 9) in place of the residues aligning with residues 189-197 of SEQ ID NO:1.

The polypeptide of the first aspect may contain a mutation of the residue aligning with residue L211 of SEQ ID NO:1.

The polypeptide of the first aspect may contain one, two, three, four, five, six, seven, eight, nine or ten of the following mutations, in any combination: N189D, A190S, P191S, R192G, S193T, S194I, M195T, S196G, N197D, L211A.

The polypeptide of the first aspect may contain the sequence DSSGTITGD (SEQ ID NO: 9) in place of the residues aligning with residues 189-197 of SEQ ID NO:1.

The polypeptide of the first aspect may have the sequence according to SEQ ID NO: 7.

### Polynucleotides, vectors, host cells, methods for producing a CTA-like polypeptide

In a second aspect, the present invention provides a polynucleotide encoding a CTA-like polypeptide according to the first aspect.

In a third aspect, the present invention provides a vector comprising a polynucleotide according to the second aspect. The vector may be an expression vector or a suicide vector.

In a fourth aspect, the present invention provides a host cell comprising a polynucleotide according to the second aspect, or a vector according to the third aspect. The host cell may be a bacterial cell, preferably a *Vibrio cholerae* cell.

Preferably, the host cells of the fourth aspect express a polynucleotide according to the second aspect.

In a fifth aspect, the present invention provides a method for producing a CTA-like polypeptide according to the first aspect, comprising the steps of:
a. providing a host cell according to the fourth aspect, expressing a polynucleotide according to the second aspect;
b. culturing said host cell in conditions such that the polypeptide is produced by the host cell; and
c. recovering said produced polypeptide unit from the culture, preferably from the culture medium.

### Adjuvants

The CTA-like polypeptide of the first aspect has utility as an adjuvant as shown in the appended Examples. Said polypeptide is less toxic than the wild-type CTA while having comparable efficacy as an adjuvant.

In order for a CTA-like polypeptide to be effective as an adjuvant, the polypeptide needs to be associated with a unit promoting its cellular entry into antigen-presenting cells.

Thus, the in the sixth aspect, the present invention provides an adjuvant with low toxicity, comprising
a cholera toxin A subunit (CTA)-like polypeptide according to the first aspect, associated with
a unit promoting cellular entry of said CTA-like polypeptide into antigen-presenting cells.

The unit promoting cellular entry may be any molecule accomplishing the task of facilitating entry of the CTA-like polypeptide into relevant cells.

Association between the CTA-like polypeptide and the unit promoting cellular entry may be covalent or non-covalent, association with CTB being an example of the latter.

Several factors known to promote cellular entry of CTA or CTA-like polypeptides into antigen-presenting cells are known in the art, and the present invention contemplates combinations of any of the known factors together with the novel CTA-like polypeptide of the first aspect.

One example of is a protein A derivative called DD, disclosed e.g. in Ågren et al. J Immunol 1997 158:3936-46.

The unit promoting cellular entry may also be a GM₁ ganglioside-binding polypeptide unit.

The GM₁-ganglioside binding polypeptide unit may be a polypeptide which is immunologically cross-reactive with antibodies raised against CTB (SEQ ID NO: 2) or LTB (SEQ ID NO: 4).

The GM₁-ganglioside binding polypeptide unit may have at least 80% sequence identity to CTB (SEQ ID NO: 2) or LTB (SEQ ID NO: 4), preferably 90%, more preferably 95%, most preferably 99%.

The GM₁-ganglioside binding polypeptide unit may be CTB.

The adjuvant according to the sixth aspect may be for use in therapy or prophylaxis, preferably for use as an adjuvant in protective immunization, most preferably in protective immunization against infection or cancer.

### Toxicity of the adjuvant

The adjuvant of the sixth aspect possesses low toxicity on mucosal administration to a mammal, meaning that the residual toxicity amounts to at least 10-fold lower toxicity compared to wild-type cholera toxin on a molar basis. Preferably, said low toxicity amounts to at least 20-fold lower toxicity compared to wild-type cholera toxin on a molar basis. More preferably, said low toxicity amounts to at least 100-fold lower toxicity compared to wild-type cholera toxin on a molar basis.

There are several ways of assessing toxicity, both *in vitro* and *in vivo.*

Toxicity of the adjuvant comprising the CTA-like polypeptide in association with CTB or other appropriate protein promoting cellular entry of the CTA-like polypeptide may be defined and measured with reference to analysing cyclic AMP production in mammalian cells or tissues, whereby the cyclic AMP production by the adjuvant of the sixth aspect is at least 10-fold lower, preferably 20-fold lower, more preferably 100-fold lower and most preferably 1000-fold lower than of wild-type cholera toxin, on a molar basis.

Several models for measuring cyclic AMP production are available in the art, for example the measurement can be made *in vitro* in mouse thymocytes as disclosed herein (see Materials and Methods).

Another way of measuring toxicity is to determine intestinal fluid secretion in an animal after intragastric or intraintestinal administration in either infant or adult mice, whereby the fluid accumulation is by the adjuvant of the sixth aspect is at least 10-fold lower, preferably 20-fold lower, and most preferably 100-fold lower than of wild-type cholera toxin, on a molar basis.

Several models for measuring intestinal fluid secretion are available in the art, for example the measurement can be made in infant mice as disclosed herein (see Materials and Methods).

### Efficacy of the adjuvant

The adjuvant according to the sixth aspect is efficacious as adjuvant, as demonstrated by the Examples. The efficacy may be defined and measured with relation to effects on oral administration to a mammal. In such model, effective adjuvant activity may amount to at least 10% adjuvant activity compared to an equimolar dose of wild-type cholera toxin, preferably at least 20%, more preferably at least 30%, most preferably at least 50% of that of an equimolar dose of wild-type cholera toxin.

The effective adjuvant activity may be measured in terms of quantitative antibody formation in response to oral *in vivo* administration of a suitable antigen together with the adjuvant e.g. as disclosed herein in Example 5.

### Compositions and vaccines of the invention

In a seventh aspect, the present invention provides a composition comprising a CTA-like polypeptide according to the first aspect and a pharmaceutically acceptable excipient, carrier or diluent. The composition may be a vaccine, further comprising an adjuvant, an antigen and/or an antigen epitope. The antigen may be an antigen from an infectious agent including but not limited to gastrointestinal pathogens such as e.g. enterotoxigenic *E. coli* or *a Vibrio cholerae.*

In an eighth aspect, the present invention provides a vaccine comprising an adjuvant according to the sixth aspect, or a composition according to the seventh aspect.

The vaccine according to the eighth aspect may further comprise an antigen from an enterotoxigenic *E. coli* or a *Vibrio cholerae.*

The vaccine according to the eighth aspect may be for use in mucosal administration. The mucosal administration may be by oral, sublingual, intragastric or rectal administration, by intranasal or pulmonary administration, by cervical or vaginal application, or by eye drops. The mucosal administration is preferably nasal or oral or sublingual administration.

The vaccine of the eighth aspect may be for use in eliciting protective immunity against a pathogen. The pathogen is preferably an enteric pathogen such as enterotoxigenic *E. coli, Vibrio cholerae, Shigella* or *Helicobacter pylori.*

Also disclosed is a use of a polypeptide according to the first aspect in a vaccine.

In a ninth aspect, the present invention provides a method for eliciting an antibody response against an antigen in a subject, comprising administering to the subject an adjuvant according to the sixth aspect and an antigen to which an antibody response is desired. The administration is preferably mucosal.

In a tenth aspect, the present invention provides a method for eliciting a cellular immune response against an antigen in a subject, comprising administering to the subject an adjuvant according to the sixth aspect and an antigen to which a cellular immune response is desired. The cellular immune response may include eliciting either or both of CD4 and CD8 T cell proliferation and induction of cytotoxic lymphocytes. Administration is preferably mucosal.

### Additional methods for producing a CTA-like polypeptide

The present invention enables production of a range of proteins in a *Vibrio cholerae-*host, including proteins that do not fall within the scope of the first aspect.

Thus, in a tenth aspect, there is provided a method for production of a reduced toxicity CTA-like polypeptide in a *Vibrio cholerae*-host, comprising the steps of:
a. providing a polynucleotide encoding for a polypeptide having at least 70 % sequence identity to cholera toxin A subunit (CTA, SEQ ID NO: 1), said polypeptide containing one or more mutations in its sequence rendering the trypsin cleavage site between amino-acids 192 and 193 of CTA trypsin-resistant and the HAP cleavage site between amino-acids 197 and 198 of CTA HAP-resistant;
b. introducing said polynucleotide to a suitable *Vibrio cholerae* host cell such that the polypeptide is expressed by the host cell;
c. culturing said host cell in conditions such that the polypeptide is produced by the host cells; and
d. recovering said produced polypeptide from the culture, in particular from the culture medium;
wherein the produced CTA-like polypeptide has reduced toxicity due to resistance to proteolytic activation.

Preferably the encoded protein has at least 75% sequence identity to wild-type CTA, more preferably at least 80% and most preferably at least 90% sequence identity to wild-type CTA.

The trypsin-resistance and HAP-resistance are defined as for the polypeptide of the first aspect.

The trypsin-resistance and HAP-resistance may be due to mutations in the primary structure corresponding to any of those described for the polypeptide of the first aspect.

The host cells may concomitantly express a polypeptide capable of associating with the CTA-like polypeptide and promoting cellular entry of the CTA-like polypeptide into antigen-presenting cells, such as a GM₁-ganglioside binding polypeptide capable of associating with said produced polypeptide. The GM₁-ganglioside binding polypeptide may a polypeptide which is immunologically cross-reactive with antibodies raised against CTB (SEQ ID NO: 2) or LTB (SEQ ID NO: 4), or may have at least 90% sequence identity to CTB (SEQ ID NO: 2) or LTB (SEQ ID NO: 4). Preferably, the GM₁-ganglioside binding polypeptide unit is CTB.

The word "comprising" is to be understood in the broad sense, as including, but not being limited to.

### Examples

The invention is further illustrated in the following working Examples. The Examples below are not to be construed as limiting.

For further experimental details, the skilled reader is referred to the Materials and Methods section that follows this section.

### Example 1: Construction of MS1405 and MS1559 and production of dmCT and mmCT

The two strains were constructed as described in Material and Methods and the insertion of the mutant ctxA genes could be confirmed by PCR analysis. Furthermore production of holotoxins could be confirmed by analysis of culture medium using GM1 ELISA in conjunction with a CTA-specific monoclonal antibody, CT17. Both strains produced CT derivatives at the same level as the parental strain 569B produced native CT.

The sequence of the entire MS1405 genome confirmed the presence of the dm*ctxA* gene at both loci expected in classical strains of O1 *V. cholerae.* Analysis of MS1559 by sequencing of PCR products showed that the strain carried the predicted mmCT sequence.

### Example 2: Analysis of the mutant cholera toxins: Generation of bacterial protease and trypsin resistant mmCT

In order to determine the properties of the mutant CTs produced by the two strains the toxins were partially purified from the growth medium of the respective strains grown up in syncase medium at 30° C by precipitation with sodium hexametaphosphate as described. The precipitates were dissolved in PBS and analysed by western blotting. The samples were compared with similar samples of native CT obtained from the parental strain 569B.

The results are shown in **figure 1**. As seen in the figure, native CT is naturally cleaved in the growth medium by bacterial proteases and when the proteins are separated by SDS-PAGE the cleaved product, CTA1 is clearly visible as the major species of CTA that reacts with the monoclonal antibody CT17. When dmCT was purified from strain MS 1405 it was shown that despite a greater proportion of CTA being non-cleaved a significant amount of cleavage still occurred.

Incubation with the partially purified dmCT with trypsin demonstrated that the modified toxin was not sensitive to tryptic digestion suggesting that there was an additional cleavage site that was sensitive to cleavage bacterially produced proteases. In order to investigate this further the cleaved dmCTA was resolved on an SDS-PAGE gel and following staining with Coomassie blue, the band was extracted and sent for analysis by mass spectrometry. The results showed that the molecule was cleaved at position 196, four residues downstream from the disrupted trypsin cleavage site.

A comparison of the LTA and CTA amino acid sequences showed that the two molecules shared 81% identity (Fig 10B). However the single region where similarity was least was in the region between the two cysteines at positions 187 and 199. In order to generate a molecule that might be insensitive to bacterial protease cleavage and yet retain the ability of the molecule to assemble properly, the region in CTA between the two cysteines at positions 187 and 199 was substituted with that from LTA.

The resulting molecule was the "multiple mutant (mm)" CTA molecule produced by the V. cholerae strain MS1559 (SEQ ID NO: 7). Analysis by western blot showed that this molecule was completely insensitive to cleavage by bacterial proteases (**figure 2**).

### Example 3: mmCT has dramatically reduced in vivo enterotoxicity and in vitro CAMP inducing activity

The relevant reduction of toxicity of mmCT in relation to CT can be determined by several different methods. One commonly used relevant method measure the enterotoxic activity leading to intestinal fluid secretion in an animal such as infant or adult mice, and other even more sensitive methods measure cAMP production in sensitive mammalian cells such as e.g. mouse thymocytes.

**Infant mouse enterotoxicity tests.** Baby mice are highly susceptible to both infection with live V. cholerae and to intragastric (i.g.)-oral exposure to cholera toxin (CT); at 18 hours after exposure to 1 microgram of CT there is intense fluid loss from the intestine resulting in swollen, edematous intestinal tissue leading to death within another 10-20 hours. To examine the enterotoxicity of mmCT compared to CT and the completely non-toxic cholera toxin B subunit (CTB) three day old infant mice (C57/BL6) were i.g. administered either 1 microgram CT (n = 10), 10 microgram mmCT (n = 9), 10 microgram CTB (n = 10) or buffer (PBS) (n = 8). After 18h the mice were weighed individually and following sacrifice intestines were examined for fluid accumulation by weighing the intestines and the remaining carcass and calculating the ratio as a measure of relative intestinal fluid accumulation. The results are shown in **Figure 3**. Significantly higher fluid accumulation was observed for CT (P < 0.001, ***) as compared with all of the other three groups when analysed by one-way-ANOVA with Bonferroni's post-test compensation for multiple analyses, and mmCT did not induce any increased fluid accumulation whatsoever and as such did not differ from either CTB or buffer only.

**Induction of cyclic AMP production.** Mouse thymocytes have turned out to be exceptionally sensitive cells to cholera toxin-induced enzymatic activity leading to ADP ribosylation of adenylate cyclase and as a result increased cyclic AMP (cAMP) production after exposure to as little as picogram amounts of CT. To test the extent of reduction in enzymatic activity of mmCT compared to CT, mouse thymocytes were exposed to different concentrations of the two proteins and the cAMP production determined by a commercial kit [Parameter cAMP determination assay (R&D systems Ltd., Abingdon, UK] Results are shown in **figure 4** and demonstrate that although mmCT can produce low amounts of cAMP at the higher concentrations used, but its activity is reduced approximately 100,000-fold in comparison with native CT.

*We conclude that mmCT has dramatically reduced in vivo enterotoxicity and in vitro cAMP inducing activity.*

### Example 4: mmCT has strong in vivo adjuvant activity enhancing both systemic and mucosal antibody responses as well as CD4 and CD8 T cell responses

The adjuvant activity of mmCT was tested in different ways and compared with that of native CT. The ability of the adjuvant to enhance antibody responses to a mucosally administered model protein antigen was tested, as was the ability of the adjuvant to enhance antigen-specific CD4⁺ T cell division in draining lymph nodes and CD8⁺ cytotoxic lymphocytes (CTLs). We did not notice any adverse reactions after any of the immunizations undertaken whether with antigen alone or together with the tested dosages of mmCT (or for that matter CT, as the dose given was a dose known to be safe for wild type CT).

**Enhancement of systemic antibody responses**. Mice were immunized intranasally (i.n.) or intragastrically (i.g.) with a model protein antigen, ovalbumin (OVA), given alone or together with mmCT or CT as described in Methods. Sera were collected after immunization and examined for IgG antibody levels. The results are shown in **figure 5** and demonstrate that both when given orally/i.g. or nasally mmCT significantly (100-fold or more) enhanced the anti-OVA antibody response and to the same antibody levels as achived with the same dose of CT.

**Enhancement of CD4⁺ T cell division.** An important function of many adjuvants is to promote antigen presentation to T cells and thus enhance the induction of mainly CD4 T cells that can both serve as helper cells for both antibody and cellular immune responses but also in some cases as effector cells. A useful method to study adjuvant activity of candidate agents, such as mmCT, is to examine their ability to promote CD4 T cell division in draining lymph nodes after immunization. To examine this for mucosally administered mmCT using a commonly used protein, ovalbumin (OVA), as model antigen, mice were first adoptively transferred with OVA-specific OT-II CD4⁺ T cells labelled ex vivo with CFSE, and one day later the mice were immunized intranasally (i.n.) with PBS, OVA, OVA + CT, or OVA + mmCT as described in Methods. Three days later the mice were sacrificed, and cervical lymph node CD4 lymphocytes isolated and examined for their extent of cell division by flow cytometry as described.

The results are shown in **figure 6** and demonstrate that while as expected the PBS-administered control mice exhibited <5% cell CD4 T cell division, the OVA-immunized mice had a significant proportion of divided OVA-specific such cells which was, however, further much increased by the co-administration of mmCT. The enhancement of antigen(OVA)-specific CD4 T cell division by mmCT was fully comparable to that achieved by the same dose of CT.

**Induction of cytotoxic lymphocytes (CTLs).** When CD8 positive T cells are activated by antigen they may develop into antigen-specific cytotoxic effector cells (CTLs) which cells are especially important in immune defense against many intracellular pathogens including both bacteria, viruses and parasites and also regarded to be of importance in immune defense against many forms of cancer. An important function for adjuvants could therefore be to be able to promote also the development of antigen-specific CD8 CTLs in response to vaccination.

The ability of mmCT to induce antigen-specific CTLs was examined in mice that had been immunized intranasally (i.n.) with PBS, OVA, OVA + CT, or OVA + mmCT as described in Methods. The results are shown in **figure 7** and demonstrate that while immunization with OVA alone under these conditions did not elicit any detectable CTL activity, the co-administration of mmCT to the same extent as native CT elicited a strong antigen(OVA)-specific CTL response.

*We conclude that mmCT has strong in vivo adjuvant activity enhancing both systemic and mucosal antibody responses as well as CD4 and CD8 T cell responses without any noticeable adverse reactions in the examined animals.*

### Example 5: Immunization of mice with a killed whole cell preparation of Vibrio cholerae with mmCT as adjuvant.

**Enhancement of intestinal mucosal IgA antibodies to cholera vaccine**. In another experiment it was examined whether mmCT could also enhance the intestinal mucosal IgA antibody response to an oral vaccine, the internationally widely licensed oral cholera vaccine Dukoral^{®}. Immunizations were given i.g. with Dukoral^{®} alone or given together with mmCT or CT as described in Methods, and after completed immunizations the mice were sacrificed and perfused with a heparin-PBS solution to remove blood from the tissues, whereafter small intestinal tissue was collected and extracted. The IgA antibody contents in the small intestinal tissue extracts against V. cholerae O1 LPS and whole-cell protein antigen were determined since such intestinal extract IgA antibodies are known to mainly if not exclusively reflect locally produced intestinal antibodies. The results are shown in **figure 8** and demonstrate that mmCT strongly enhanced the fecal IgA anti-cholera antibody response induced by the Dukoral^{®} vaccine to both LPS and to the protein antigen.

### Materials and Methods

### Bacterial strains and plasmids.

The O1 classical *Vibrio cholerae* strain JS1569 is a rifampicin resistant derivative of the *ctxA* deleted strain CVD103. This strain was used to generate the strains MS1405 and MS1559 used for the production of the mutant CT derivatives.

The *E. coli* strain S17-1 was used for the maintenance and propagation of suicide plasmids used for the construction of the mutant CT producing strains MS1405 and MS1559.

All strains were maintained on Luria-Bertani (LB) agar plates supplemented when necessary with appropriate antibiotics (chloramphenicol 12.5 µg/ml, rifampicin 50 µg/ml) and were stored at -70° C in medium containing 17% glycerol.

Small liquid cultures (5 ml) of *E. coli* S17-1 for routine extraction of plasmid were grown at 37° C in LB broth supplemented with chloramphenicol with rotary shaking at 180 rpm.

25 ml pre-cultures for production of protein were grown overnight in LB-broth at 37° C with shaking (180 rpm). These were used to inoculate flasks (1:100) containing 500 ml fresh syncase medium which were incubated at 30° C with shaking (180 rpm) for 20-24h.

The suicide plasmid pMT-suicide/sacB was generated in this laboratory. The plasmid is a derivative of pMT-suicide (Lebens et al. Vaccine. 2011 ; 9(43):7505-13) in which the sacB gene of Bacillus subtilis has been inserted in order to provide a counter-selection of loss of the plasmids from transconjugants.

Primers for the generation of the ctxA gene encoding mmCTA were:
1) LT/CTHybl: 5'-GCAAGTATCACCTGTAATTGTTCCTGATGAATCCCCACAACCCGGCGGTGCATGATGAATCC-3' (SEQ ID NO: 12)
2) LT/CTHyb3:5'-GATTCATCAGGAACAATTACAGGTGATACTTGCGATGAAAAAACCCAAAGTCTAGGTGTAAAATTCG CTG-3' (SEQ ID NO: 13)
3) pMT primer A: 5'-GGCGCCCATGGTGAAAACGGGGGCGAAG-3' (SEQ ID NO: 14)
4) pMT primer B: 5'-GGCGCCCATGGGCAAATATTATACGCAAGGCGAC-3' (SEQ ID NO: 15)

The primer combinations for the first amplification were primers 1+3 and 2+4.

For amplification of the entire plasmid following primerless PCR primer combination 3+4 was used.

### DNA manipulation

All DNA maniputations were performed using enzymes and reagents obtained from Thermo Fischer Scientific Inc., MA, USA. All reactions were carried out using buffers supplied by the manufacturer under recommended reaction conditions.

All DNA primers were obtained from Eurofins MGW Operon (Ebersberg, Germany) who also provided sequencing services. The longer double-stranded sequence used for generating the first CTA mutant described below was obtained from ATG-Biosynthetics GmbH (Freiburg, Germany).

Genomic sequencing was done in as part of a larger project collaboration with the Sanger Institute, Hixton, UK.

### GM1 ELISA for toxin assays

The presence and approximate concentrations of the toxins was determined by GM1 ELISA as previously described (A M Svennerholm and G Wiklund J. Clin. Microbiol. 1983, 17(4):596). The primary antibodies used were LT39 (Svennerholm AM, Wikström M, Lindblad M, Holmgren J. Med Biol. (1986)64: 23-30) specifically recognizing CTB and CT17 specifically recognizing CTA. The secondary antibody used was a goat anti-mouse IgG-HRP conjugate obtained from SouthernBiotech (Birmingham, AL, USA).

### Construction of V. cholerae strains MS1405 and MS1559

The O1 Inaba classical *V. cholerae* strains MS1405 and MS1559 were generated by reinsertion of the deleted *ctxA* gene in the parental strain JS1569. In MS1405 the *ctxA* gene carried the mutations R192G and L211A similar to those in the previously described dmLT (Norton EB, Lawson LB, Freytag LC, Clements JD. Clin Vaccine Immunol. 2011 Apr;18(4):546-51, SEQ ID NO: 5). In MS1559 in addition to the two mutations in MS1405 there are mutations in the region 189 to 197. In dmCTA the sequence is NAPGSSMSN (SEQ ID NO: 8) whereas in mmCTA the sequence is DSSGTITGD (SEQ ID NO: 9). The underlined residue in each sequence is the R192G mutation present in both molecules.

The reinsertion of the mutant *ctxA* genes was done by gene replacement using the above described suicide plasmid pMT-suicide/sacB. A native *ctxA* gene together with a fragment of the upstream *zot* gene was PCR amplified from chromosomal DNA isolated as described previously from strain Phil6973 ; An O1 El tor clinical isolate of *V. cholerae* using the primers zot f (5'-GGGGGTCTCTCTAGAATGCTGCGGGAGCAAGGCGGCTG-3', SEQ ID NO: 10) and CTA r (5'-GGGGGGAAGCTTATAATTCATCCTTAATTCTATTATGTGTATCAATATCAGATTG-3' (SEQ ID NO 11) (see table 1). The resulting fragment was digested with Eco31I and Hindlll and inserted into a p15A-based expression vector digested with Xbal and HindIII. In order to generate the double mutant CTA with amino acid changes R192G and L211A, a DNA fragment was synthesized the allowed the entire 3' end of the gene to be substituted between a unique BspEI site in the *ctxA* gene and the terminal HindIII site. The changes resulted in the loss of the unique ClaI site in the *ctxA* gene and its replacement with a unique BamHI site which was subsequently used to screen for positive clones by restriction analysis. The sequence of positive clones was confirmed by sequence analysis.

The expression plasmid containing the double mutant *ctxA* gene was then transferred into the *V. cholerae* strain JS1569 containing a compatible pMB1-based expression vector carrying the *ctxB* gene. With both plasmids present in the same background, liquid cultures were grown under inducing conditions. Resulting culture supernatants were then assayed by GM1 ELISA for the presence of holotoxin using the CTA-specific monoclonal antibody CT17. This demonstrated that the dmCTA was expressed and assembled with CTB giving similar yields to strains expressing native CT in the same manner. The mutated *ctxA* gene was then linked to a previously cloned recombinant *ctxB* gene to regenerate a *ctxAB* operon containing the double mutant *ctxA* gene. The entire operon was then cloned into the suicide vector pMT-suicide/sacB as an Xbal/Xhol fragment resulting in the plasmid pMT-ssBdmCT.

The second modified *ctxA* molecule was generated from pMT-ssBdmCT using PCR to generate the further modifications. Two fragments encompasing the entire plasmid were amplified. Essentially two of the primers contain overlapping sequences containing the additional mutations. These together with the other primers were used to amplify the entire plasmid in two halves. The resulting fragments were then linked together using primerless PCR which resulted in a full length plasmid carrying the mutant *ctxA* gene. This could be digested with Ncol and ligated to obtain a circular plasmid that could be transformed into *E. coli* strain S17-1. The sequence of the *ctxAB* operon was confirmed by DNA sequencing.

Primers for the generation of the ctxA gene encoding mmCTA were:
1) LT/CTHyb1: 5'-GCAAGTATCACCTGTAATTGTTCCTGATGAATCCCCACAACCCGGCGGTGCATGATGAATCC-3' (SEQ ID NO: 12)
2) LT/CTHyb3:5'-GATTCATCAGGAACAATTACAGGTGATACTTGCGATGAAAAAACCCAAAGTCTAGGTGTAAAATTCG CTG-3' (SEQ ID NO: 13)
3) pMT primer A: 5'-GGCGCCCATGGTGAAAACGGGGGCGAAG-3' (SEQ ID NO: 14)
4) pMT primer B: 5'-GGCGCCCATGGGCAAATATTATACGCAAGGCGAC-3' (SEQ ID NO: 15)

The primer combinations for the first amplification were primers 1+3 and 2+4.

For amplification of the entire plasmid following primerless PCR primer combination 3+4 was used.

Both constructs were then used to generate *V. cholerae* strains expressing the mutant CT molecules. Briefly, plasmids were transferred into the recipient strain JS1569 by conjugation with selection for colonies resistant to chloramphenicol and rifampicin. Resulting strains were then checked for production of holotoxin since the introduced fragment carried the native promoter allowing normal expression of CT. Positive clones were chosen for further development. These were passaged in liquid LB broth over a period of five days before plating out serial dilutions on LB agar plates containing no salt and supplemented with 8% sucrose. Single colonies obtained in this manner were then screened for clones that had lost the plasmid (become sensitive to chloramphenicol) and retained the ability to produce CT.

Resulting colonies with the correct phenotype were analyzed using PCR in order to check for correct insertion of the mutant *ctxA* genes into the chromosomes of *V. cholerae* in the appropriate positions.

Strains with the correct sequences expressing the double mutant (dm)CT and the multiple mutated (mm)CT were called MS1405 and MS1559 respectively.

Finally the sequence of the entire genome of the strain MS1405 was determined and compared with that of the parental strain 569B.

### Protein production and analysis.

The different proteins were produced from the respective *V. cholerae* strains cultured as described above. Native CT was produced from the parental O1 classical strain 569B. dmCT and mmCT were produced from strains MS1405 and MS1559 respectively. After overnight growth the cells were removed from the growth medium by centrifugation at 7,000 x g for 15 minutes. The cells were discarded and the medium was sterilized by filtration though a 0.22 µm filter. The toxins were then precipitated by the addition of 2.5 mg/ml of sodium hexametaphosphate and adjusting the pH to 4.5 as described previously (Lebens M, Johansson S, Osek J, Lindblad M, Holmgren J. Biotechnology (N Y). 1993 Dec;11(13):1574-8). The precipitates were collected by centrifugation and re-dissolved in a minimal volume of 50mM Tris-HCl pH 7.5. Non-dissolved material was removed by centrifugation followed by filtration though a 0.22 µm filter and the resulting solution subjected to anion exchange chromatography using a Resource Q anion exchange column connected to an ÄKTA FPLC apparatus (GE Healthcare Life Sciences). Proteins were eluted using a sodium chloride gradient (0-0.5 M). Fractions were analyzed by SDS-PAGE analysis. When necessary a final purification step was performed using gel filtration. Crude protein preparations that were not subjected ion exchange or gel filtration were used to analyze cleavage of the A subunit of the different holotoxins. This was done by SDS-PAGE followed by Western blotting and detection of CTA with the CT17 monoclonal antibody described above. The secondary antibody was the same as that used for GM1 ELISA and the bands were detected using ortho-chloronaphthol as described previously (A M Svennerholm and G Wiklund J. Clin. Microbiol. 1983, 17(4):596).

### Toxicity analysis.

Preliminary toxicity analysis of the mmCT was done using an assay measuring intestinal fluid accumulation in infant mice. Primigravida female C57BL/6N mice were purchased from Charles River Laboratories (Sweden). Three days after birth infant mice weighing between 2.3 and 2.7 grams were separated from their mothers, randomly grouped and placed at 26°C for 4 hours. Each mouse was thereafter intragastrically inoculated with 50 µl using a sterile feeding needle, and immediately placed back at 26°C. After 18 hours the animals were one by one weighed, and sacrifice. Thereafter the small intestine (pyloric valve to ileal-cecal junction) was removed in one piece and weighed. Fluid accumulation (FA) was calculated using the formula FA = [small intestinal weight/carcass weight] × 10³. All animals in the study were treated and housed under specific-pathogen-free conditions in accordance to the Swedish Animal Welfare Act (1988:534) and the Animal Welfare Ordinance (1988:539). Approval for the study was given by the Ethical Committee for Laboratory Animals in Gothenburg, Sweden.

### Enzymatic activity of mmCT.

In order to analyse the ADP ribosylating enzymatic activity of the mmCT molecule the production of cyclic AMP (cAMP) was measured in mouse thymocytes following intoxication with purified toxin. Sample preparation and determination of cAMP levels by ELISA was done in accordance with manufacturer's instructions (R&D Systems), with some modifications. Briefly, single cell suspension of thymocytes (5x106) were treated with various concentrations of CT, mmCT, dmLT, and CTB, or left untreated. Following incubation at 37 °C with 5% CO2 for 2 ½ hours, cells were washed 3 times with cold PBS, suspended in 500 µl of 1x cell lysis buffer, and stored at -20 °C. Two additional freeze/thaw cycles was done, and complete lysis of cells was confirmed by trypan blue exclusion. Cell debris was then removed by centrifugation at 600 x g for 5 minutes, with cell lysates stored at -20 °C. For the cAMP assay procedure, microplate strips were initially added with 50 µL of primary antibody solution for 1-hour incubation at RT in a horizontal orbital microplate shaker (500 rpm). Following washing 4x with wash buffer, wells were added with 100 ul of the samples or twofold serial dilutions of the standard, plus 50 µL of cAMP conjugate for 2-hour incubation at RT with shaking. After 4x washing, wells were incubated with 200 µL of substrate for 30 minutes at RT in the dark, and later added with 100 µL of stop solution. Optical density was determined within 30 minutes using a microplate reader set at 450 nm. The standard curve was plotted with a four parameter logistic curve-fit, which set the basis for calculating the concentration of cAMP in the samples.

### Assessment of adjuvant activity of mmCT

The adjuvant activity of mmCT was tested in different ways. The ability of the adjuvant to enhance the antibody response to a mucosally administered model protein antigen was tested, as was the ability of the adjuvant to enhance antigen-specific CD4⁺ T cell division in draining lymph nodes and CD8⁺ cytotoxic lymphocytes (CTLs). Further, the ability of mmCT to enhance antibody responses to a killed whole cell cholera vaccine was investigated.

### Studies of antibody responses.

For studies of antibody responses, female C57 BI/6 mice (Charles River Laboratories, Willmington, MA; 6-8 weeks of age; 6 mice per group) were mucosally immunized either intranasally (i.n.) with ovalbumin (OVA) or intragastrically (i.g.) with the Dukoral™ oral cholera vaccine alone or together with mmCT or CT. Two or three rounds of immunization were given at 12-15 days intervals. For the i.n. administrations each dose of OVA (10 microgram) with or without mmCT or CT (2 microgram) were given in a 10 microliter volume by a pipette in one nostril. The i.g. administrations were given in 0.3 ml 3% (w/v) sodium bicarbonate using oral gavage through a baby feeding catheter, with each dose being divided in three parts administered on consecutive days with food removed for 2-3 hours before each administration. Mice used as negative controls were given buffer only.

Bleedings were performed and sera prepared before the first, and for the i.n. immunized mice 20 days and for the i.g. immunized mice 10-12 days after the last round of immunization at which time-points fecal pellets were also collected and extracted as described (). Specific IgG antibodies against OVA in serum, and specific IgA antibodies against *V. cholerae* O1 lipopolysaccharide (LPS) or whole cell lysate antigen preparations in small intestinal tissue extracts from heparin-perfused sacrificed mice (obtained by the so-called PerFext method) were determined by ELISA. Antibody titers were expressed as the reciprocals of the extrapolated sample dilutions which gave an A₄₅₀ absorbance of 0.4 above background.

### CD4+ T cell division studies.

Mice were adoptively transferred by an intravenous injection with 5 x 10⁶ OVA-specific OT-II CD4⁺ T cells labelled ex vivo with 4 µM CFSE; one day later the mice were immunized intranasally (I.N.) with a single dose of PBS, OVA, OVA + CT, or OVA + mmCT; the amounts used were 10 µg OVA with or without 2µg CT or mmCT administered in a total volume of 10 µl. Three days later the mice were sacrificed and cervical lymph nodes collected and CD4⁺ lymphocytes isolated and examined for cell division by analysing their extent of CFSE staining by flow cytometry as described.

### CTL responses.

C57BL/6 mice were immunized intranasally (I.N.) with PBS, OVA, OVA + CT, or OVA + mmCT; the doses used were 5 µg OVA with or without 1.5µg CT or mmCT in a total volume of 10 µl. Immunized mice were one week later injected i.v. with 8 x 10⁶ 57BL/6 splenocytes. Donor splenocytes at a 1:1 ratio of cells pulsed with OT-I-specific peptide OVA₂₅₇₋₂₆₄ (SINFEKL; 2µg/ml) and stained with 4 µM CFSE (high CFSE dose cohort), and non-pulsed cells stained with 0.4 µM CFSE (low CFSE dose cohort). One day after adoptive transfer splenocytes were analyzed for presence of CFSE-labeled cells by flow cytometry. The percent specific lysis was determined by loss of the peptide-pulsed CFSE^{high} population compared with the unpulsed cFSE^{low} population using the formula: 100-(ratio in experimental mouse /ratio in naïve mouse x 100).

### Immunization of mice with a killed whole cell preparation of Vibrio cholerae.

BALB/C female mice (7 mice per group) were orally immunized with the licensed cholera vaccine Dukoral with and without the addition of mmCT as adjuvant.

The volume final volume of vaccine administered was 300 µL containing 2.5x10⁹ bacteria. This dose was repeated on consecutive days giving a total dose of 5x109 bacteria per immunization round. When mmCT was added the dose was 10 µg per administration and therefore 20 µg per immunization round.

The mice were treated with two immunization rounds two weeks apart.

Ten days following the second immunization round the mice were sacrificed and blood, fecal samples and small intestine were taken for analysis.

Antibodies were perfused from the intestinal samples using saponin extraction.

Immune (specifically IgA) responses against *V. cholerae* lipopolysaccharide (LPS) and soluble proteins were determined by ELISA.

In both cases it was seen that there was a significant increase in the *V. cholerae*-specific IgA responses in the mice that received mmCT.

### Statistical analyses.

Unless otherwise indicated, statistical analyses between groups were conducted by Student's two-tailed *t*-test using Prism software, with *P* values of <0.05 regarded as significant.

## Claims

1. A cholera toxin A subunit (CTA)-like polypeptide having at least 90% sequence identity to CTA (SEQ ID NO: 1), **characterized in that**:
a. the CTA-like polypeptide contains one or more mutations in its sequence rendering the trypsin cleavage site between amino-acids 192 and 193 of CTA trypsin-resistant; and
b. the CTA-like polypeptide contains one or more mutations in its sequence rendering the *Vibrio cholerae* HAP cleavage site between amino-acids 197 and 198 of CTA HAP-resistant.

2. The polypeptide according to claim 1, wherein trypsin-resistance and HAP-resistance are defined such that the trypsin and HAP cleavage sites are cleaved at least 100-fold slower by trypsin and HAP, respectively, compared to corresponding sites in wild-type CTA (SEQ ID NO: 1) under corresponding conditions.

3. The polypeptide according to any of the preceding claims, wherein the polypeptide comprises one or more mutations in amino-acid residues aligning with residues 189-200 of SEQ ID NO:1, compared to SEQ ID NO: 1.

4. The polypeptide according to any of the preceding claims, wherein the polypeptide comprises an amino acid sequence having at least 95 % identity to SEQ ID NO: 1.

5. The polypeptide according to any of the preceding claims, wherein the polypeptide contains a mutations at the residues aligning with
a. residues R192 and N197 of SEQ ID NO:1, or
b. residues R192 and T198 of SEQ ID NO:1.

6. The polypeptide according to any of the preceding claims, wherein the polypeptide has the sequence according to SEQ ID NO: 7.

7. An adjuvant with low toxicity, comprising
a. a cholera toxin A subunit (CTA)-like polypeptide according to any of claims 1-6, associated with
b. a unit promoting cellular entry of said CTA-like polypeptide into antigen-presenting cells.

8. The adjuvant according to claim 7, wherein the unit promoting cellular entry is a protein A derivative termed DD or a GM₁ ganglioside-binding polypeptide unit.

9. The adjuvant according to claim 8 wherein the unit promoting cellular entry is a GM₁-ganglioside binding polypeptide unit being a polypeptide which is immunologically cross-reactive with antibodies raised against CTB (SEQ ID NO: 2) or LTB (SEQ ID NO: 4), or having at least 90% sequence identity to CTB (SEQ ID NO: 2) or LTB (SEQ ID NO: 4).

10. The adjuvant according to claim 9 wherein the unit promoting cellular entry is a GM₁-ganglioside binding polypeptide unit being CTB (SEQ ID NO: 2).

11. A vaccine comprising an adjuvant according to any of claims 7-10.

12. The vaccine according to claim 11, further comprising an antigen from an enterotoxigenic *E. coli* or *Vibrio cholerae.*

13. A host cell comprising a polynucleotide encoding a CTA-like polypeptide according to any one of claims 1-6.

14. A method for production of a reduced toxicity CTA- like polypeptide in a *Vibrio cholerae*-host, comprising the steps of:
a. providing a polynucleotide encoding for a polypeptide having at least 70 % sequence identity to cholera toxin A subunit (CTA, SEQ ID NO: 1), said polypeptide containing one or more mutations in its sequence rendering the trypsin cleavage site between amino-acids 192 and 193 of CTA trypsin-resistant and the HAP cleavage site between amino-acids 197 and 198 of CTA HAP-resistant;
b. introducing said polynucleotide to a suitable *Vibrio cholerae* host cell such that the polypeptide is expressed by the host cell;
c. culturing said host cell in conditions such that the polypeptide is produced by the host cells; and
d. recovering said produced polypeptide from the culture; wherein the produced CTA-like polypeptide has reduced toxicity due to resistance to proteolytic activation.

15. The method according to claim 10, wherein the CTA-like polypeptide has mutations in relation to SEQ ID NO: 1 as defined in any one of claims 3-6.
